(19) Europäisches Patentamt
European Patent Office
Office européen des brevets

(11) EP 4 230 225 A1

(12) **EUROPEAN PATENT APPLICATION**
published in accordance with Art. 153(4) EPC

(43) Date of publication:
**23.08.2023 Bulletin 2023/34**

(21) Application number: **21879564.9**

(22) Date of filing: **19.10.2021**

(51) International Patent Classification (IPC):
***A61K 47/68*** *(2017.01)* ***A61K 39/395*** *(2006.01)*
***C07K 16/18*** *(2006.01)* ***C07K 16/30*** *(2006.01)*
***A61P 35/00*** *(2006.01)*

(52) Cooperative Patent Classification (CPC):
**A61K 38/07; A61K 39/395; A61K 45/00; A61K 45/06; A61K 47/68; A61P 1/00; A61P 1/18; A61P 35/00; C07K 16/18; C07K 16/30**

(86) International application number:
**PCT/CN2021/124698**

(87) International publication number:
**WO 2022/078523 (21.04.2022 Gazette 2022/16)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.10.2020 CN 202011105383**

(71) Applicants:
- **Shanghai Miracogen Inc.**
  **Shanghai 201203 (CN)**
- **Keymed Biosciences Co.,Ltd.**
  **Chengdu, Sichuan 610219 (CN)**

(72) Inventors:
- **HU, Chaohong**
  **Shanghai 201203 (CN)**
- **LI, Hu**
  **Shanghai 201203 (CN)**
- **CHEN, Bo**
  **Chengdu, Sichuan 610219 (CN)**
- **XU, Gang**
  **Chengdu, Sichuan 610219 (CN)**
- **WANG, Ying**
  **Chengdu, Sichuan 610219 (CN)**

(74) Representative: **Mewburn Ellis LLP**
**City Tower**
**40 Basinghall Street**
**London EC2V 5DE (GB)**

Remarks:
- A request for restoration of the right of priority by the EPO as designated Office has been granted (R. 49ter.2 PCT, Art.122 EPC).
- The complete document including Reference Table(s) and the Sequence Listing(s) can be downloaded from the EPO website

(54) **ANTIBODY-DRUG CONJUGATE AND APPLICATION THEREOF**

(57) The present invention relates to an antibody-drug conjugate and an application thereof, and specifically provides the antibody-drug conjugate, a pharmaceutically acceptable salt and solvate thereof, or a solvate of the salt. The antibody-drug conjugate has the structure represented by formula I, wherein Ab is an anti-Claudin 18.2 antibody. The antibody-drug conjugate of the present invention has good tumor cell growth inhibition activity in vivo and in vitro, low toxicity, and good application prospects.

$$\text{Ab-}(\text{L-D})_p \qquad \text{Formula I}$$

EP 4 230 225 A1

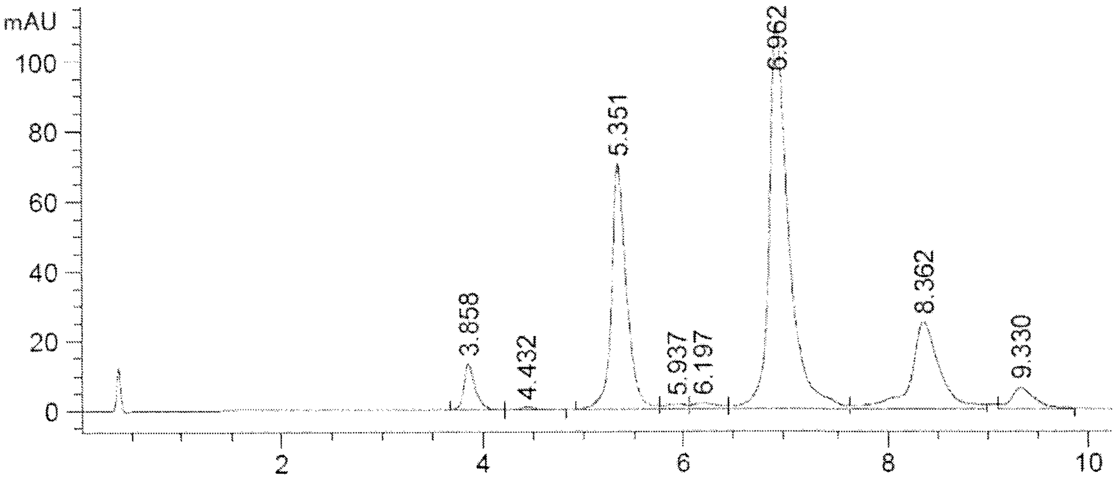
mAU
100
80
60
40
20
0
3.858
4.432
5.351
5.937
6.197
6.962
8.362
9.330
2
4
6
8
10

Fig. 6

## Description

Technical Field

The present invention relates to the field of medicinal chemistry, in particular, to antibody drug conjugates and applications thereof.

Background technique

Gastric cancer is one of the most common cancers worldwide, with higher incidences in East Asia, Eastern Europe, and South America, and lower incidences in North America and Africa. The standard initial treatment for advanced or recurrent gastric cancer is chemotherapy. Although the prognosis of gastric cancer patients has been improved significantly due to the development of surgical techniques and perioperative treatment, the 5-year overall survival rate is only 10-15%. Targeted therapy has brought new hope for the treatment of recurrent/advanced gastric cancer. Trastuzumab combined with chemotherapy can bring some benefits to HER2 positive patients, but only 15% of patients have HER2 positive expression, and the beneficiary population is limited. In recent years, immunotherapy has brought new hope for the treatment of recurrent/advanced gastric cancer; however, according to the results of the KEYNOTE-12 study, the proportion of PD-L1 positive populations suitable for this treatment only accounts for 40% of patients with recurrent/advanced gastric cancer or adenocarcinoma of the esophagogastric junction. Therefore, the development of new gastric cancer treatment drugs is still imminent.

SUMMARY OF THE INVENTION

The inventor of the present application has prepared an anti-Claudin 18.2 antibody drug conjugate through a lot of experiments and creative work, and confirmed that it has good biological activity, thus completing the present invention.

To this end, in the first aspect of the present invention, the present invention provides an antibody drug conjugate, a pharmaceutically acceptable salt, solvate or solvate of said salt, and the antibody drug conjugate has the structure shown in Formula I,

$$Ab\text{-}(L\text{-}D)_p \qquad \text{Formula I}$$

wherein:

Ab is an anti-Claudin 18.2 antibody which comprises a heavy chain and a light chain, and the heavy chain variable region CDR1 comprises a sequence selected from a sequence as shown in SEQ ID NO: 2, 10, 18, 26, 34, 42, 68, 76, 84 , 92, 100, 108 or 116 or a mutant thereof, the heavy chain variable region CDR2 comprises a sequence selected from a sequence as shown in SEQ ID NO: 3, 11, 19, 27, 35, 43, 69, 77, 85, 93, 101, 109 or 117 or a mutant thereof, and the heavy chain variable region CDR3 comprises a sequence selected from a sequence as shown in SEQ ID NO: 4, 12, 20, 28, 36, 44, 70, 78, 86, 94,102, 110 or 118 or a mutant thereof, the light chain variable region CDR1 comprises a sequence selected from a sequence as shown in SEQ ID NO: 50, 58, 124 or 132 or a mutant thereof, the light chain variable region CDR2 comprises a sequence selected from a sequence as shown in SEQ ID NO: 51, 59, 125 or 133 or a mutant thereof, and the light chain variable region CDR3 comprises a sequence selected from a sequence as shown in SEQ ID NO: 52, 60,126 or 134 or a mutant thereof;
D is a cytotoxic agent.
L is a linker for linking the anti-Claudin 18.2 antibody and the cytotoxic agent;
p is 2.0-8.0 (e.g., 2.0-7.0, 2.0-6.0, 2.0-5.0, 2.0-4.0, 3.0-7.0, 3.0-6.0, 3.0-5.0 or 3.0-4.0, or e.g., 3.0, 4.0, 5.0, 6.0 or 7.0).

The antibody drug conjugate of the present invention has good tumor cell growth inhibition activity both *in vivo* and *in vitro,* and has a good application prospect. The antibody-drug conjugate of the present invention is formed by linking the anti-Claudin 18.2 antibody and dolastatin derivative MMAE through the MC-vc-PAB linker, and its anti-tumor mechanism of action is as follows. After binding to Claudin 18.2 on the surface of the tumor cells, the antibody drug conjugate enters tumor cells through endocytosis and transports to lysosomes, and then it is degraded by proteases in the lysosomes to release MMAE. After entering the cytoplasm, MMAE binds to tubulin and inhibits its polymerization, thereby blocking various physiological functions of cells including mitosis tubulin involved with, thereby inhibiting tumor cell proliferation and leading to tumor cell death.

It should be noted that the "antibody drug conjugate" is a composition containing ADC molecules with the same or different DAR values. Specifically, the present invention provides compositions comprising a plurality of ADC molecules. In certain instances, the multiple ADCs each comprise the same number of drug molecules in the composition. In other instances, the multiple ADCs each comprise a different number of drug molecules in the composition.

The above drug-to-antibody ratio (DAR) refers to the number of drug molecules (e.g., p in Formula I) conjugated to the antibody. The number of drug molecules contained in the antibody-drug conjugate of the present invention (for example, p in Formula I) is usually an integer. When the number of drug molecules contained in the antibody-drug conjugate of the present invention (e.g., p in Formula I) is a fraction, it refers to the average number of drug molecules conjugated per antibody in a composition comprising a plurality of ADC molecules.

The above drug-to-antibody ratios (DAR) can be verified by conventional means, such as mass spectrometry, ELISA assays, HIC and HPLC. The quantitative distribution of ADCs with respect to p can also be determined. In some cases, the separation, purification and validation of a homogeneous ADC for which p is a certain value from ADCs with other drug loads can be accomplished by means such as reverse phase HPLC or electrophoresis.

In some embodiments, the heavy chain variable region CDR1 of the anti-Claudin 18.2 antibody comprises a sequence selected from a sequence as shown in SEQ ID NO: 2, 10, 18, 26, 34 or 42 or a mutant thereof, the heavy chain variable region CDR2 comprises a sequence selected from a sequence as shown in SEQ ID NO: 3, 11, 19, 27, 35 or 43 or a mutant thereof, the heavy chain variable region CDR3 comprises a sequence selected from a sequence as shown in SEQ ID NO: 4, 12, 20, 28, 36 or 44 or a mutant thereof, the light chain variable region CDR1 comprises a sequence selected from a sequence as shown in SEQ ID NO: 50 or 58 or a mutant thereof, the light chain variable region CDR2 comprises a sequence selected from a sequence as shown in SEQ ID NO: 51 or 59 or a mutant thereof, and the light chain variable region CDR3 comprises a sequence selected from a sequence as shown in SEQ ID NO: 52 or 60 or a mutant thereof.

In some embodiments, the heavy chain variable region CDR1 of the anti-Claudin 18.2 antibody comprises a sequence selected from a sequence as shown in SEQ ID NO: 68, 76, 84, 92, 100, 108 or 116 or a mutant thereof, the heavy chain variable region CDR2 comprises a sequence selected from a sequence as shown in SEQ ID NO: 69, 77, 85, 93, 101, 109 or 117 or a mutant thereof, the heavy chain variable region CDR3 comprises a sequence selected from a sequence as shown in SEQ ID NO: 70, 78, 86, 94, 102, 110 or 118 or a mutant thereof, the light chain variable region CDR1 comprises a sequence selected from a sequence as shown in SEQ ID NO: 124 or 132 or a mutant thereof, the light chain variable region CDR2 comprises a sequence selected from a sequence as shown in SEQ ID NO: 125 or 133 or a mutant thereof, and the light chain variable region CDR3 comprises a sequence selected from a sequence as shown in SEQ ID NO: 126 or 134 or a mutant thereof.

In some embodiments, the heavy chain variable region CDR1, CDR2, CDR3 of the anti-Claudin 18.2 antibody are selected from the following sequence combinations:

(1) SEQ ID NO: 2, SEQ ID NO: 3, SEQ ID NO: 4,
(2) SEQ ID NO: 10, SEQ ID NO: 11, SEQ ID NO: 12,
(3) SEQ ID NO: 18, SEQ ID NO: 19, SEQ ID NO: 20,
(4) SEQ ID NO: 26, SEQ ID NO: 27, SEQ ID NO: 28,
(5) SEQ ID NO: 34, SEQ ID NO: 35, SEQ ID NO: 36,
(6) SEQ ID NO: 42, SEQ ID NO: 43, SEQ ID NO: 44,
(7) SEQ ID NO: 68, SEQ ID NO: 69, SEQ ID NO: 70,
(8) SEQ ID NO: 76, SEQ ID NO: 77, SEQ ID NO: 78,
(9) SEQ ID NO: 84, SEQ ID NO: 85, SEQ ID NO: 86,
(10) SEQ ID NO: 92, SEQ ID NO: 93, SEQ ID NO: 94,
(11) SEQ ID NO: 100, SEQ ID NO: 101, SEQ ID NO: 102,
(12) SEQ ID NO: 108, SEQ ID NO: 109, SEQ ID NO: 110,
(13) SEQ ID NO: 116, SEQ ID NO: 117, SEQ ID NO: 118;

the light chain variable region CDR1, CDR2, CDR3 of the anti- claudin 18.2 antibody are selected from the following sequence combinations:

(1) SEQ ID NO: 50, SEQ ID NO: 51, SEQ ID NO: 52,
(2) SEQ ID NO: 58, SEQ ID NO: 59, SEQ ID NO: 60,
(3) SEQ ID NO: 124, SEQ ID NO: 125, SEQ ID NO: 126,
(4) SEQ ID NO: 132, SEQ ID NO: 133, SEQ ID NO: 134.

In some embodiments, the heavy chain variable region CDR1, CDR2, CDR3 of the anti-Claudin 18.2 antibody are selected from the following sequence combinations:

(1) SEQ ID NO: 2, SEQ ID NO: 3, SEQ ID NO: 4,

(2) SEQ ID NO: 10, SEQ ID NO: 11, SEQ ID NO: 12,
(3) SEQ ID NO: 18, SEQ ID NO: 19, SEQ ID NO: 20,
(4) SEQ ID NO: 26, SEQ ID NO: 27, SEQ ID NO: 28,
(5) SEQ ID NO: 34, SEQ ID NO: 35, SEQ ID NO: 36,
(6) SEQ ID NO: 42, SEQ ID NO: 43, SEQ ID NO: 44;

the light chain variable region CDR1, CDR2 and CDR3 of the anti-Claudin 18.2 antibody are selected from the following sequence combinations:

(1) SEQ ID NO: 50, SEQ ID NO: 51, SEQ ID NO: 52,
(2) SEQ ID NO: 58, SEQ ID NO: 59, SEQ ID NO: 60.

In some embodiments, the heavy chain variable region CDR1, CDR2, CDR3 of the anti-Claudin 18.2 antibody are selected from the following sequence combinations:

(1) SEQ ID NO: 68, SEQ ID NO: 69, SEQ ID NO: 70,
(2) SEQ ID NO: 76, SEQ ID NO: 77, SEQ ID NO: 78,
(9) SEQ ID NO: 84, SEQ ID NO: 85, SEQ ID NO: 86,
(4) SEQ ID NO: 92, SEQ ID NO: 93, SEQ ID NO: 94,
(5) SEQ ID NO: 100, SEQ ID NO: 101, SEQ ID NO: 102,
(6) SEQ ID NO: 108, SEQ ID NO: 109, SEQ ID NO: 110,
(7) SEQ ID NO: 116, SEQ ID NO: 117, SEQ ID NO: 118;

the light chain variable region CDR1, CDR2 and CDR3 of the anti-Claudin 18.2 antibody are selected from the following sequence combinations:

(1) SEQ ID NO: 124, SEQ ID NO: 125, SEQ ID NO: 126,
(2) SEQ ID NO: 132, SEQ ID NO: 133, SEQ ID NO: 134.

In some embodiments, the heavy chain variable region FR1 of the anti-Claudin 18.2 antibody comprises a sequence selected from a sequence as shown in SEQ ID NO: 5, 13, 21, 29, 37, 45, 71, 79, 87, 95, 103, 111 or 119 or a mutant thereof, the heavy chain variable region FR2 comprises a sequence selected from a sequence as shown in SEQ ID NO: 6, 14, 22, 30, 38, 46, 72, 80, 88, 96, 104, 112 or 120 or a mutant thereof, the heavy chain variable region FR3 comprises a sequence selected from a sequence as shown in SEQ ID NO: 7, 15, 23, 31, 39, 47, 73, 81, 89, 97, 105, 113 or 121 or a mutant thereof, the heavy chain variable region FR4 comprises a sequence selected from a sequence as shown in SEQ ID NO: 8, 16, 24, 32, 40, 48, 74, 82, 90, 98, 106, 114 or 122 or a mutant thereof, the light chain variable region FR1 comprises a sequence selected from a sequence as shown in SEQ ID NO: 53, 61, 127 or 135 or a mutant thereof, the light chain variable region FR2 comprises a sequence selected from a sequence as shown in SEQ ID NO: 54, 62, 128 or 136 or a mutant thereof, the light chain variable region FR3 comprises a sequence selected from a sequence as shown in SEQ ID NO: 55, 63, 129 or 137 or a mutant thereof, and the light chain variable region FR4 comprises a sequence selected from a sequence as shown in SEQ ID NO: 56, 64, 130 or 138 or a mutant thereof.

In some embodiments, the heavy chain variable region FR1 of the anti-Claudin 18.2 antibody comprises a sequence selected from a sequence as shown in SEQ ID NO: 5, 13, 21, 29, 37 or 45 or a mutant thereof, the heavy chain variable region FR2 comprises a sequence selected from a sequence as shown in SEQ ID NO: 6, 14, 22, 30, 38 or 46 or a mutant thereof, the heavy chain variable region FR3 comprises a sequence selected from a sequence as shown in SEQ ID NO: 7, 15, 23, 31, 39 or 47 or a mutant thereof, the heavy chain variable region FR4 comprises a sequence selected from a sequence as shown in SEQ ID NO: 8, 16, 24, 32, 40 or 48 or a mutant thereof, the light chain variable region FR1 comprises a sequence selected from a sequence as shown in SEQ ID NO: 53 or 61, or a mutant thereof, the light chain variable region FR2 comprises a sequence selected from a sequence as shown in SEQ ID NO: 54 or 62 or a mutant thereof, the light chain variable region FR3 comprises a sequence selected from a sequence as shown in SEQ ID NO: 55 or 63 or a mutant thereof, and the light chain variable region FR4 comprises a sequence selected from a sequence as shown in SEQ ID NO: 56 or 64 or a mutant thereof.

In some embodiments, the heavy chain variable region FR1 of the anti-Claudin 18.2 antibody comprises a sequence selected from a sequence as shown in SEQ ID NO: 71, 79, 87, 95, 103, 111 or 119 or a mutant thereof, the heavy chain variable region FR2 comprises a sequence selected from a sequence as shown in SEQ ID NO: 72, 80, 88, 96, 104, 112 or 120 or a mutant thereof, the heavy chain variable region FR3 comprises a sequence selected from a sequence as shown in SEQ ID NO: 73, 81, 89, 97, 105, 113 or 121 or a mutant thereof, the heavy chain variable region FR4 comprises a sequence selected from a sequence as shown in SEQ ID NO: 74, 82, 90, 98, 106, 114 or 122 or a

mutant thereof, the light chain variable region FR1 comprises a sequence selected from SEQ ID NO: 127 or 135 or a mutant thereof, the light chain variable region FR2 comprises a sequence selected from SEQ ID NO: 128 or 136 or a mutant thereof, the light chain variable region FR3 comprises a sequence selected from SEQ ID NO: 129 or 137 or a mutant thereof, and the light chain variable region FR4 comprises a sequence selected from SEQ ID NO: 130 or 138 or a mutant thereof.

In some embodiments, the heavy chain variable region FR1, FR2, FR3, FR4 of the anti-Claudin 18.2 antibody are selected from the following sequence combinations:

(1) SEQ ID NO: 5, SEQ ID NO: 6, SEQ ID NO: 7, SEQ ID NO: 8,
(2) SEQ ID NO: 13, SEQ ID NO: 14, SEQ ID NO: 15, SEQ ID NO: 16,
(3) SEQ ID NO: 21, SEQ ID NO: 22, SEQ ID NO: 23, SEQ ID NO: 24,
(4) SEQ ID NO: 29, SEQ ID NO: 30, SEQ ID NO: 31, SEQ ID NO: 32,
(5) SEQ ID NO: 37, SEQ ID NO: 38, SEQ ID NO: 39, SEQ ID NO: 40,
(6) SEQ ID NO: 45, SEQ ID NO: 46, SEQ ID NO: 47, SEQ ID NO: 48,
(7) SEQ ID NO: 71, SEQ ID NO: 72, SEQ ID NO: 73, SEQ ID NO: 74,
(8) SEQ ID NO: 79, SEQ ID NO: 80, SEQ ID NO: 81, SEQ ID NO: 82,
(9) SEQ ID NO: 87, SEQ ID NO: 88, SEQ ID NO: 89, SEQ ID NO: 90,
(10) SEQ ID NO: 95, SEQ ID NO: 96, SEQ ID NO: 97, SEQ ID NO: 98,
(11) SEQ ID NO 103, SEQ ID NO 104, SEQ ID NO 105, SEQ ID NO 106,
(12) SEQ ID NO: 111, SEQ ID NO: 112, SEQ ID NO: 113, SEQ ID NO: 114,
(13) SEQ ID NO: 119, SEQ ID NO: 120, SEQ ID NO: 121, SEQ ID NO: 122;

the light chain variable region FR1, FR2, FR3 and FR4 of the anti-Claudin 18.2 antibody are selected from the following sequence combinations:

(1) SEQ ID NO: 53, SEQ ID NO: 54, SEQ ID NO: 55, SEQ ID NO: 56,
(2) SEQ ID NO: 61, SEQ ID NO: 62, SEQ ID NO: 63, SEQ ID NO: 64,
(3) SEQ ID NO: 127, SEQ ID NO: 128, SEQ ID NO: 129, SEQ ID NO: 130,
(4) SEQ ID NO: 135, SEQ ID NO: 136, SEQ ID NO: 137, SEQ ID NO: 138.

In some embodiments, the heavy chain variable region FR1, FR2, FR3, FR4 of the anti-Claudin 18.2 antibody are selected from the following sequence combinations:

(1) SEQ ID NO: 5, SEQ ID NO: 6, SEQ ID NO: 7, SEQ ID NO: 8,
(2) SEQ ID NO: 13, SEQ ID NO: 14, SEQ ID NO: 15, SEQ ID NO: 16,
(3) SEQ ID NO: 21, SEQ ID NO: 22, SEQ ID NO: 23, SEQ ID NO: 24,
(4) SEQ ID NO: 29, SEQ ID NO: 30, SEQ ID NO: 31, SEQ ID NO: 32,
(5) SEQ ID NO: 37, SEQ ID NO: 38, SEQ ID NO: 39, SEQ ID NO: 40,
(6) SEQ ID NO:45, SEQ ID NO:46, SEQ ID NO:47, SEQ ID NO:48;

the light chain variable region FR1, FR2, FR3 and FR4 of the anti-Claudin 18.2 antibody are selected from the following sequence combinations:

(1) SEQ ID NO: 53, SEQ ID NO: 54, SEQ ID NO: 55, SEQ ID NO: 56,
(2) SEQ ID NO:61, SEQ ID NO:62, SEQ ID NO:63, SEQ ID NO:64.

In some embodiments, the heavy chain variable region FR1, FR2, FR3, FR4 of the anti-Claudin 18.2 antibody are selected from the following sequence combinations:

(1) SEQ ID NO:71, SEQ ID NO:72, SEQ ID NO:73, SEQ ID NO:74,
(2) SEQ ID NO: 79, SEQ ID NO: 80, SEQ ID NO: 81, SEQ ID NO: 82,
(3) SEQ ID NO: 87, SEQ ID NO: 88, SEQ ID NO: 89, SEQ ID NO: 90,
(4) SEQ ID NO: 95, SEQ ID NO: 96, SEQ ID NO: 97, SEQ ID NO: 98,
(5) SEQ ID NO: 103, SEQ ID NO: 104, SEQ ID NO: 105, SEQ ID NO: 106,
(6) SEQ ID NO: 111, SEQ ID NO: 112, SEQ ID NO: 113, SEQ ID NO: 114,
(7) SEQ ID NO: 119, SEQ ID NO: 120, SEQ ID NO: 121, SEQ ID NO: 122;

the light chain variable region FR1, FR2, FR3 and FR4 of the anti-Claudin 18.2 antibody are selected from the following

sequence combinations:

(1) SEQ ID NO: 127, SEQ ID NO: 128, SEQ ID NO: 129, SEQ ID NO: 130,
(2) SEQ ID NO: 135, SEQ ID NO: 136, SEQ ID NO: 137, SEQ ID NO: 138.

In some embodiments, the heavy chain variable region of the anti-Claudin 18.2 antibody is selected from a sequence as shown in SEQ ID NO: 1, 9, 17, 25, 33, 41, 67, 75, 83, 91, 99, 107, or 115;
the light chain variable region of the anti-Claudin 18.2 antibody is selected from a sequence as shown in SEQ ID NO: 49, 57, 123 or 131.
In some embodiments, the heavy chain variable region of the anti-Claudin 18.2 antibody is selected from a sequence as shown in SEQ ID NO: 1, 9, 17, 25, 33 or 41;
the light chain variable region of the anti-Claudin 18.2 antibody is selected from a sequence as shown in SEQ ID NO: 49 or 57.
In some embodiments, the heavy chain variable region of the anti-Claudin 18.2 antibody is selected from a sequence as shown in SEQ ID NO: 67, 75, 83, 91, 99, 107 or 115;
the light chain variable region of the anti-Claudin 18.2 antibody is selected from a sequence as shown in SEQ ID NO: 123 or 131.
In some embodiments, the heavy chain variable region and light chain variable region of the anti-Claudin 18.2 antibody are selected from the following sequence combinations:

(1) SEQ ID NO: 17 and SEQ ID NO: 57,
(2) SEQ ID NO: 41 and SEQ ID NO: 49,
(3) SEQ ID NO: 41 and SEQ ID NO: 57,
(4) SEQ ID NO:115 and SEQ ID NO:131.

In some embodiments, the sequences of the heavy chain variable region and the light chain variable region of the anti-Claudin 18.2 antibody are SEQ ID NO: 41 and SEQ ID NO: 49, respectively.
In some embodiments, the heavy chain constant region of the anti-Claudin 18.2 antibody is selected from human IgG (e.g., IgG1, IgG2, IgG3, or IgG4), IgM, IgA, IgD, IgA constant regions, or mutants of the above constant regions, preferably human IgG1;
the light chain constant region of the anti-Claudin 18.2 antibody is selected from human lambda constant region, kappa constant region or a mutant of the above constant regions, preferably a human kappa constant region.
In some embodiments, the amino acid sequence of the heavy chain of the anti-Claudin 18.2 antibody comprises the sequence set forth in SEQ ID NO:65, or a sequence having greater than 70%, such as greater than 75%, 80%, 85%, 90%, 95%, 99% identity to SEQ ID NO: 65;
the amino acid sequence of the light chain of the anti-Claudin 18.2 antibody comprises a sequence as shown in SEQ ID NO: 66, or a sequence having greater than 70%, such as greater than 75%, 80%, 85%, 90%, 95%, 99% identity to SEQ ID NO: 66.
In some embodiments, p is 3.0-4.0.
In some embodiments, p is 3.0-3.8.
In some embodiments, p is 3.0, 3.4, 3.5, or 3.8.
In some embodiments, p is 3.8.
In some embodiments, the cytotoxic agent is selected from the group consisting of SN-38, Gemcitabine, Monomethyl auristatin E (MMAE), Monomethyl auristatin F (MMAF), maytansinoids (e.g., Maytansine DM1, Maytansine DM4), calicheamicin, MGBA (e.g., duocarmycin), doxorubicin, ricin, Diphtheria toxin and other toxins, 1131, interleukins, tumor necrosis factor, chemokines and nanoparticles.
In some embodiments, the cytotoxic agent is MMAE.
The structure of MMAE is:

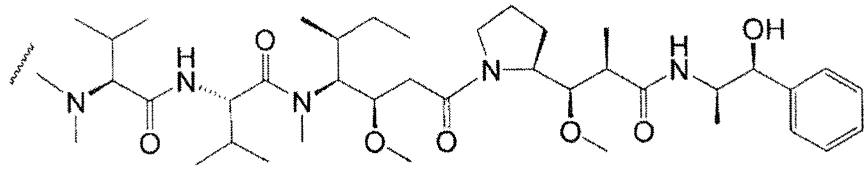

.

In some embodiments, the linker is selected from the group consisting of 6-maleimidohexanoyl (MC), maleimidopropionyl (MP), N-succinimidyl 4-(2-pyridylthio) valerate (SPP), 4-(N-maleimidomethyl)-cyclohexan-1-formyl (MCC), N-succinimidyl(4-iodo-acetyl)aminobenzoate (SIAB), and 6-maleimidocaproyl-valine-citrulline-p-aminobenzyloxycarbonyl (MC-vc-PAB).

In some embodiments, the linker is 6-maleimidocaproyl-valine-citrulline-p-aminobenzyloxycarbonyl (MC-vc-PAB).

In some embodiments, L-D as described in Formula I is MC-vc-PAB-MMAE, and its structure is shown in the following formula:

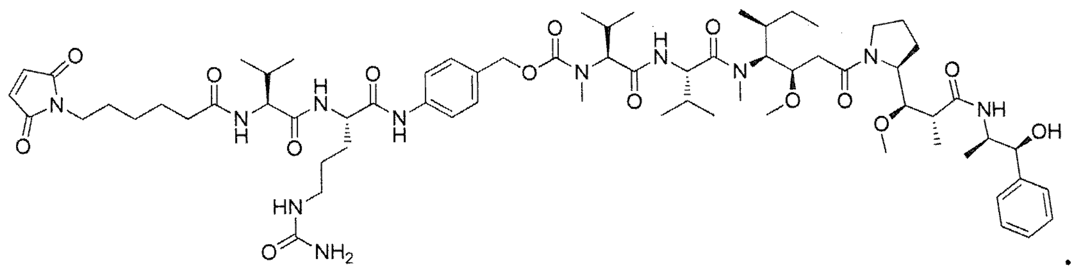

In some embodiments, Ab includes:

(a) heavy chain variable region CDR1, CDR2, CDR3 and light chain variable region CDR1, CDR2, CDR3, wherein the sequence of heavy chain variable region CDR1 is shown in SEQ ID NO: 42, the sequence of heavy chain variable region CDR2 is shown in SEQ ID NO: 43, the sequence of heavy chain variable region CDR3 is shown in SEQ ID NO: 44, the sequence of the light chain variable region CDR1 is shown in SEQ ID NO: 50, the sequence of the light chain variable region CDR2 is shown in SEQ ID NO: 51, and the sequence of light chain variable region CDR3 is shown in SEQ ID NO: 52;
(b) a heavy chain variable region and a light chain variable region, wherein the sequence of the heavy chain variable region is shown in SEQ ID NO: 41, and the sequence of the light chain variable region is shown in SEQ ID NO: 49; and /or
(c) heavy chain and light chain, wherein the sequence of the heavy chain is shown in SEQ ID NO: 65, and the sequence of the light chain is shown in SEQ ID NO: 66;

L is MC-vc-PAB; and
D is MMAE.

In a second aspect of the present invention, the present invention provides a composition comprising the aforementioned antibody drug conjugate, a pharmaceutically acceptable salt, solvate or solvate of said salt.

In some embodiments, the composition further comprises known chemotherapeutic drugs for the treatment of tumors, such as doxorubicin (Adriamycin), cyclophosphamide, taxanes [such as paclitaxel (Taxol) ), docetaxel (Taxotere)], capecitabine (Xeloda), gemcitabine (Gemzar), vinorelbine (Navelbine), tamoxifen, aromatase inhibitors (Arimidex, Furlong, Arnold New), 5-FU plus leucovorin, irinotecan (camptosar), oxaliplatin, cisplatin, carboplatin, estramustine, mitoxantrone (Novantrone), prednisone, vincristine (Oncovin), doxorubicin, prednisone, etc., or a combination thereof.

In some embodiments, the composition further comprises a known immunotherapeutic drugs for treating tumors, e.g., a PD-1 monoclonal antibody (such as pembrolizumab, nivolumab, etc.), a PD-L1 monoclonal antibody (such as Atezolizumab), a TIGIT monoclonal antibody, a 4-1BB monoclonal antibody, a VEGFR2 monoclonal antibody (such as Ramucirumab, apatinib), a HER2 monoclonal antibody (such as trastuzumab, Trastuzumab biosimilar, Trastuzumab-dkst), etc., or a combination thereof.

In some embodiments, the composition further comprises an immunosuppressant selected from: (1) glucocorticoids, such as cortisone and prednisone; (2) microbial metabolites, such as cyclosporine and tacrolimus, etc.; (3) antimetabolites, such as azathioprine and 6-mercaptopurine, etc.; (4) polyclonal and monoclonal anti-lymphocyte antibodies, such as anti-lymphocyte globulin and OKT3, etc.; (5) alkylating agents, such as cyclophosphamide. Specifically, the immunosuppressants are, for example, methylprednisolone, prednisone, azathioprine, prograf, xenipra, sule, cyclosporine, tacrolimus, rapamycin, mycophenolate mofetil, mizoribine, cyclophosphamide, fingolimod, etc.

In some embodiments, the composition further comprises a pharmaceutically acceptable carrier, diluent or excipient.

In the third aspect of the present invention, the present invention provides use of the aforementioned antibody-drug conjugate, its pharmaceutically acceptable salt, solvate or solvate of said salt or the aforementioned composition in the preparation of medicaments, said medicaments are used for the prevention and/or treatment of a disease associated

with Claudin 18.2.

In some embodiments, the disease associated with Claudin 18.2 is gastric cancer, adenocarcinoma of the esophagogastric junction, pancreatic cancer.

In some embodiments, the disease associated with Claudin 18.2 is gastric cancer.

In the fourth aspect of the present invention, the present invention provides methods for preventing and/or treating a disease associated with Claudin 18.2, comprising: administering to a subject in need thereof a prophylactically and/or therapeutically effective amount of the aforementioned antibody drug conjugate compound, its pharmaceutically acceptable salt, solvate or solvate of said salt, or the aforementioned composition.

In some embodiments, the disease associated with Claudin 18.2 is gastric cancer, adenocarcinoma of the esophagogastric junction, pancreatic cancer.

In some embodiments, the disease associated with Claudin 18.2 is gastric cancer.

In the fifth aspect of the present invention, the present invention provides the aforementioned antibody drug conjugate, its pharmaceutically acceptable salt, solvate or solvate of said salt, or the aforementioned composition, which is used for preventing and/or treating a disease associated with Claudin 18.2.

In some embodiments, the disease associated with Claudin 18.2 is gastric cancer, adenocarcinoma of the esophagogastric junction, pancreatic cancer.

In some embodiments, the disease associated with Claudin 18.2 is gastric cancer.

Description of drawings

Fig. 1 shows the RT-PCR of the Example of the present invention showing that HEK293 stably transfected cell lines express Claudin 18.1 and Claudin 18.2 respectively; HEK293 stably transfected cell lines expressing Claudin 18.2 and control KATO III cells can both amplify the 780bp characteristic band specific to Claudin 18.2, while HEK293 expressing Claudin 18.1 can only amplify a common fragment of 504bp.

Fig. 2 is the result of screening HEK293 stably transfected cell lines expressing high levels of Claudin 18.2 by FACS in the Example of the present invention, wherein the black dots are negative controls, and the gray dots are HEK293 stably transfected cell lines expressing high levels of Claudin 18.2.

Fig. 3 is the result of screening NIH3T3 stably transfected cell lines expressing high levels of Claudin 18.2 by FACS in the Example of the present invention, wherein the black line is the negative control, and the gray shade is 3T3 stably transfected cell lines expressing Claudin 18.2; NO.32-H is 3T3 stably transfected cell lines expressing high levels of Claudin 18.2, NO.18-M is 3T3 stably transfected cell lines expressing medium levels of Claudin 18.2, and NO.6-L is 3T3 stably transfected cell lines expressing low levels of Claudin 18.2.

Fig. 4 is a graph showing the results of the ADCC effect of the Anti-Claudin18.2 antibody according to the Example of the present invention.

Fig. 5 is a graph showing the results of the CDC effect of Anti-Claudin18.2 antibody according to the Example of the present invention.

Fig. 6 is the hydrophobic interaction chromatogram (HIC) of the antibody-drug conjugate according to the Example of the present invention.

Fig. 7 is a graph showing the results of the cell killing effect on LT-M11 cell line of different CM311 ADCs according to the Example of the present invention.

Fig. 8 is a graph showing the results of the inhibitory activity on the tumor growth of human gastric cancer nude mouse PDX model STO#025 of CM311-ADC-1 and control CM311-ADC-2 according to the Example of the present invention.

Fig. 9 is a graph showing the effects on the body weight of animals in the PDX model of human gastric cancer nude mice STO#025 of CM311-ADC-1 and control CM311-ADC-2 according to the Example of the present invention.

Fig. 10 is a graph showing the results of the inhibitory activity on the tumor growth of human gastric cancer nude mouse PDX model STO#523 of CM311-ADC-1 and control CM311-ADC-2 according to the Example of the present invention.

Fig. 11 is a graph showing the effect on the body weight of animals in the PDX model of human gastric cancer nude mice STO#523 of CM311-ADC-1 and control CM311-ADC-2 according to the Example of the present invention.

Fig. 12 is a graph showing the comparison results of the in vitro cell activity of the antibody-drug conjugate according to the Example of the present invention and the antibody (QC Log transformation independent fitting graph).

Specific Embodiments

The embodiments of the present invention will be described in details with reference to the following examples,

and it will be understood by those skilled in the art the following examples are intended to be illustrative of the invention and are not to be taken as limiting the scope of the present invention. Where specific conditions are not indicated in the examples, they are carried out according to the conventional conditions or the conditions suggested by the manufacturer. The reagents or instruments used, where the manufacturer is not specified, are conventional products that could be obtained from the market.

In the present invention, unless otherwise specified, scientific and technical terms used herein have the meanings commonly understood by those skilled in the art. Moreover, the protein and nucleic acid chemistry, molecular biology, cell and tissue culture, microbiology, immunology related terms and laboratory procedures used herein are the terms and routine procedures widely used in the corresponding fields. Meanwhile, for a better understanding of the present invention, definitions and explanations of related terms are provided below.

Claudin protein is a skeletal protein that constitutes a tight junction structure. It is located on the top side of the adjacent intercellular spaces. Its distribution is tissue-organ-specific. Its main functions are intercellular adhesion, maintenance of cell polarity, regulation of paracellular permeability, and participation in regulation of cell proliferation and differentiation. In tumors, the tight junctions between cells are destroyed and Claudin cannot perform its normal function.

Claudin 18 is a member of the Claudin family with 2 different first exons, so two isoforms can be generated by alternative splicing: Claudin 18.1 and Claudin 18.2. These two isoforms are transcribed and amplified in different tissues respectively, among them, Claudin 18.1 is mainly expressed in lung tissue, while Claudin 18.2 is specifically expressed in gastric tissue. Claudin 18.2 (GenBank accession number: NM _001002026.3) is not expressed in other normal tissues except gastric mucosa, but it is significantly up-regulated in various tumors, including 80% of gastrointestinal adenomas, 60% of pancreas tumors, and some tumors of the bile ducts, ovaries, and lungs.

The term "Claudin 18.2-related disease" refers to a disease in which the expression of Claudin 18.2 in tissue cells differs from (e.g., exceeds) the normal levels. For example, if the expression level of Claudin 18.2 in certain tissue cells is higher than the expression level of Claudin 18.2 in the reference or control (i.e., normal tissue cells), it indicates that the existence of Claudin 18.2-related diseases in the object (especially the human) from which the tissue cells are derived.

In the present invention, unless otherwise indicated, any numerical range should be understood to include any value or any sub-range within the range.

In the present invention, the term "antibody" refers to an immunoglobulin molecule generally composed of two identical pairs of polypeptide chains, each pair having one "light" (L) chain and one "heavy" (H) chain. The light chains of antibodies can be divided into two categories: kappa and lambda. Heavy chains can be divided into five types: $\mu$, $\delta$, $\gamma$, $\alpha$ or $\varepsilon$, and antibodies can be divided into five types: IgM, IgD, IgG, IgA and IgE according to the difference of the heavy chain. Within the light- and heavy chains, the variable and constant regions are linked by a "J" region of about 12 or more amino acids, and the heavy chain also contains a "D" region of about 3 or more amino acids. Each heavy chain is composed of a heavy chain variable region ($V_H$) and a heavy chain constant region ($C_H$). The heavy chain constant region consists of 3 domains ($C_H1$, $C_H2$ and $C_H3$). Each light chain is composed of a light chain variable region ($V_L$) and a light chain constant region ($C_L$). The light chain constant region consists of one domain, $C_L$. The constant regions of the antibodies could mediate the binding of the immunoglobulin to host tissues or factors, including various cells of the immune system (e.g., effector cells) and component C1q of the complement system. The $V_H$ and $V_L$ regions can also be subdivided into regions of high variability called complementarity determining regions (CDRs) interspersed with more conserved regions called framework regions (FRs). Each $V_H$ and $V_L$ consists of 3 CDRs and 4 FRs arranged in the following order: FR1, CDR1, FR2, CDR2, FR3, CDR3, FR4 from amino terminus to carboxy terminus. The variable regions ($V_H$ and $V_L$) of each heavy/light chain pair, respectively, form the antibody binding site. The assignment of amino acids to respective regions or structural domains follows the definition of Kabat Sequences of Proteins of Immunological Interest (National Institutes of Health, Bethesda, Md. (1987 and 1991)), or Chothia & Lesk (1987) J. Mol. Biol. 196:901-917, Chothia et al. (1989) Nature 342:878-883.

In the present invention, algorithms for determining sequence identity (homology) and percent sequence similarity are, for example, the BLAST and BLAST 2.0 algorithms, respectively described in Altschul et al. (1977) Nucl. Acid. Res. 25:3389- 3402 and Altschul et al. (1990) J. Mol. Biol. 215:403-410. BLAST and BLAST 2.0 can be used to determine percent of amino acid sequence identity of the present invention using, for example, those described in the literature or default parameters. Software to perform BLAST analyses is available to the public through the National Center for Biotechnology Information.

In the present invention, the amino acid sequence having at least 70% sequence identity to the amino acid sequence includes a polypeptide sequence that is substantially identical to the amino acid sequence, e.g., those which contain at least 70% sequence identity, preferably at least 75%, 80%, 85%, 86%, 87%, 88%, 89%, 90%, 91%, 92%, 93%, 94%, 95%, 96%, 97%, 98%, 99% or higher sequence identity compared to the polypeptide sequence of the invention, when using the methods described herein (e.g., BLAST analysis using standard parameters).

In the present invention, the mutant of the amino acid sequence refers to the one which has identity of more than 70%, such as more than 75%, 80%, 85%, 90%, 91%, 92%, 93%, 94%, 95%, 96%, 97%, 98%, 99% to the amino

acid sequence, e.g., sequences with 3, 2 or 1 substitution, deletion or addition of amino acids. Preferably, no more than 3 amino acids are substituted, added or deleted. More preferably, no more than 2 amino acids are substituted, added or deleted. Most preferably, no more than 1 amino acid is substituted, added or deleted.

A "substitutional" variant is one in which at least one amino acid residue in the native sequence has been removed and a different amino acid inserted in its same position. The substitutions can be single, wherein only one amino acid is substituted in the molecule, or multiple, wherein the same molecule has two or more amino acids substituted. Multiple substitutions can be made at consecutive sites. Likewise, one amino acid may be substituted by multiple residues, wherein such variants include both substitutions and insertions. An "insertion" (or "additive") variant is one in which one or more amino acids are inserted into a particular position immediately adjacent to a native sequence. Immediately adjacent to an amino acid means attachment to the alpha-carboxyl or alpha-amino functional group of the amino acid. A "deletion" variant is one in which one or more amino acids in the native amino acid sequence have been removed. Typically, deletion variants have one or two amino acids deleted in a specific region of their molecule.

In certain embodiments, less than the theoretical maximum of the drug moiety is conjugated to the antibody in the conjugation reaction. In general, antibodies do not contain many free and reactive cysteine thiol groups that can link drug moieties; in fact, most cysteine thiol groups in antibodies exist as disulfide bridges. In certain embodiments, the antibody can be reduced with a reducing agent such as dithiothreitol (DTT) or tris(2-carboxyethyl)phosphine (TCEP) under partially or fully reducing conditions to generate reactive cysteine thiol groups.

In some embodiments, the pharmaceutically acceptable salt is an inorganic acid salt or an organic acid salt, wherein the inorganic acid salt is hydrochloride, hydrobromide, hydroiodide, nitrate, bicarbonate, carbonate, sulfate or phosphate, the organic acid salt is formate, acetate, propionate, benzoate, maleate, fumarate, succinate, tartrate, citrate, ascorbate, $\alpha$-ketoglutarate, $\alpha$-glycerophosphate, alkyl sulfonate or aryl sulfonate; preferably, the alkyl sulfonate is methanesulfonate or ethanesulfonate; the aryl sulfonate is benzenesulfonate or p-toluenesulfonate.

Pharmaceutically acceptable salts can be obtained using standard procedures well known in the field, for example, by reacting a sufficient amount of a basic compound with a suitable acid which provides a pharmaceutically acceptable anion.

As used herein, unless otherwise specified, the term "prodrug" refers to a derivative that can be hydrolyzed, oxidized, or otherwise reacted under biological conditions (in vitro or in vivo) to provide a compound of the present invention. Prodrugs only undergo this reaction under biological conditions to become the active compounds, or they are unactive in their unreacted form. Prodrugs can generally be prepared using well-known methods, such as those described in Burger's Medicinal Chemistry and Drug Discovery (1995) 172-178, 949-982 (Manfred E. Wolff, ed., 5th ed.).

In the present invention, solvates refer to these forms of the antibody-drug conjugates of the present invention: complexes in solid or liquid form formed by coordination of the antibody-drug conjugates with solvent molecules. Hydrates are a specific form of solvates, which have coordinated water molecules. In the present invention, hydrates are the preferred solvates.

Methods of preparing various pharmaceutical compositions containing amounts of active ingredients are known, or will be apparent to those skilled in the art in light of the present disclosure. As described in REMINGTON'S PHARMACEUTICAL SCIENCES, Martin, E.W., ed., Mack Publishing Company, 19th ed. (1995), methods of preparing such pharmaceutical compositions include incorporating suitable pharmaceutical excipients, carriers, diluents, etc., which are not toxic to cells or mammals when they are exposed to at the doses and concentrations employed.

The pharmaceutical composition of the present invention may comprise a pH buffered aqueous solution; alternatively may comprise, buffers, such as phosphates, citrates, and other organic acids; antioxidants, including ascorbic acid; low molecular weight (less than about 10 residues) polypeptides; proteins, such as serum albumin, gelatin, or immunoglobulins; hydrophilic polymers such as polyvinylpyrrolidone; amino acids, such as glycine, glutamine, asparagine, arginine or lysine; monosaccharides, disaccharides and other carbohydrates, including glucose, mannose, sucrose, trehalose or dextrin; chelating agents such as EDTA; sugar alcohols such as mannitol or sorbitol; salt-forming counterions such as sodium; and/or non-ionic surfactants such as TWEN™, polyethylene glycol (PEG) and PLURONICS™.

The pharmaceutical formulations of the present invention are manufactured by known methods, including conventional mixing, dissolving or lyophilization methods. The compounds of the present invention can be formulated into pharmaceutical compositions and administered to patients by various routes suitable for the chosen mode of administration, for example, orally or parenterally (by intravenous, intramuscular, topical or subcutaneous routes).

Thus, the compounds of the present invention in combination with a pharmaceutically acceptable carrier (e.g., an inert diluent or an assimilable and edible carrier) can be administered systemically, for example, orally. They can be enclosed in hard- or soft-shell gelatin capsules, which can be compressed into tablets. For oral therapeutic administration, the active compound may be incorporated with one or more excipients and presented in the form of swallowable tablets, buccal tablets, lozenges, capsules, elixirs, suspensions, syrups, wafers, and the like. Such compositions and preparations should contain at least 0.1% active compound. The proportions of such compositions and formulations may, of course, vary and may range from about 1% to about 99% by weight of a given unit dosage form. In such therapeutically useful

compositions, the active compound is in an amount such that an effective dosage level can be obtained.

Tablets, troches, pills, capsules, etc. may also contain: binders, such as tragacanth, acacia, cornstarch, or gelatin; excipients, such as dicalcium hydrogen phosphate; disintegrants, such as cornstarch, potato starch, alginic acid, etc.; lubricants, such as magnesium stearate; and sweeteners, such as sucrose, fructose, lactose, or aspartame; or flavoring agents, such as peppermint, oil of wintergreen, or cherry flavor. When the unit dosage form is a capsule, it could contain, in addition to materials of the above type, a liquid carrier such as a vegetable oil or polyethylene glycol. Various other materials may be present, as coatings, or otherwise alter the physical form of the solid unit dosage form. For example, tablets, pills or capsules could be coated with gelatin, wax, shellac or sugar and the like. A syrup or elixir could contain the active compounds, sucrose or fructose as a sweetening agent, methyl or propyl paraben as a preservative, a dye and a flavoring (such as cherry flavor or orange flavor). Of course, any materials used in the preparation of any unit dosage form should be pharmaceutically acceptable and substantially non-toxic in the amounts to be used. In addition, the active compounds can be incorporated into sustained release formulations and sustained release devices.

The active compounds could also be administered intravenously or intraperitoneally by infusion or injection. Aqueous solutions of the active compounds or salts thereof could be prepared, optionally mixed with nontoxic surfactants. Dispersions could also be prepared in glycerol, liquid polyethylene glycols, triacetin, and mixtures thereof and in oils. Under ordinary conditions of storage and use, these preparations contain a preservative to prevent the growth of microorganisms.

Pharmaceutical dosage forms suitable for injection or infusion may include sterile aqueous solutions or dispersions or sterile powder containing the active ingredient (optionally encapsulated in liposomes) suitable for extemporaneous preparation in sterile injectable or infusible solutions or dispersions. In all cases, the final dosage form must be sterile, liquid, and stable under the conditions of manufacture and storage. The liquid carrier can be a solvent or liquid dispersion medium containing, for example, water, ethanol, polyol (e.g., glycerol, propylene glycol, liquid polyethylene glycol, and the like), vegetable oils, nontoxic glycerides, and suitable mixtures thereof. Proper fluidity can be maintained, for example, by the formation of liposomes, by the maintenance of the desired particle size in the case of dispersions, or by the use of surfactants. Prevention of microorganisms can be brought about by various antibacterial and antifungal agents such as parabens, chlorobutanol, phenol, sorbic acid, thimerosal, and the like. In many cases it is preferred to include isotonic agents such as sugars, buffers or sodium chloride. Prolonged absorption of the injectable compositions can be brought about by the use of agents which delay absorption, for example, aluminum monostearate and gelatin.

Sterile injectable solutions are prepared by incorporating the active compounds in the required amount in an appropriate solvent with various of the other ingredients enumerated above as required, followed by filtered sterilization. In the case of sterile powders for the preparation of sterile injectable solutions, the preferred methods of preparation are vacuum drying and freeze-drying techniques, which yield a powder of the active ingredient plus any additional required ingredients previously present in sterile-filtered solutions.

Useful solid carriers include pulverized solids (e.g., talc, clays, microcrystalline cellulose, silica, alumina, and the like). Useful liquid carriers include water, ethanol or ethylene glycol, or water-ethanol/ethylene glycol mixtures, in which the compounds of the present invention could be dissolved or dispersed at effective levels, optionally with the aid of non-toxic surfactants. Adjuvants (e.g., fragrances) and additional antimicrobial agents can be added to optimize properties for a given use.

Thickeners (such as synthetic polymers, fatty acids, fatty acid salts and esters, fatty alcohols, modified celluloses or modified inorganic materials) could also be used with liquid carriers to form spreadable pastes, gels, ointments, soap, etc., which could be used directly on the user's skin.

The above formulations may be presented in unit dosage form, which are physically discrete units containing unitary dosages, suitable for administration to the human and other mammalian bodies. The unit dosage form can be a capsule or tablet, or a number of capsules or tablets. Depending on the particular treatment involved, the amount of active ingredient in a unit dose may vary or be adjusted from about 0.1 to about 1000 mg or more.

In addition, it also includes the application of various new drug dosage forms such as lacto-liposomes, microspheres and nanospheres, such as the use of microparticle dispersion systems including polymeric micelles, nanoemulsions, submicroemuls, microcapsules, microspheres, liposomes and niosomes (also known as non-ionic surfactant vesicles).

The term "treating" as used herein generally refers to obtaining a desired pharmacological and/or physiological effect. The effect may be prophylactic in terms of complete or partial prevention of the disease or its symptoms; and/or therapeutic in terms of partial or complete stabilization or cure of the disease and/or side effects due to the disease. "Treatment" as used herein encompasses any treatment of a disease in a patient, including: (a) prevention of disease or symptoms in a patient susceptible to a disease or condition but not yet diagnosed; (b) suppression of symptoms of disease, i.e., preventing its development; or (c) alleviating the symptoms of the disease, i.e., causing the disease or symptoms to regress.

In the present invention, "subject" refers to a vertebrate. In certain embodiments, vertebrate refers to a mammal. Mammals include, but are not limited to, livestock (such as cattle), pets (such as cats, dogs, and horses), primates, mice,

and rats. In certain embodiments, the mammal refers to a human.

In the present invention, "effective amount" refers to an amount effective to achieve the desired therapeutic or prophylactic effect at the necessary dose and time. The "therapeutically effective amount" of a substance/molecule of the present invention may vary depending on factors such as the disease state, age, sex and weight of the individual and the ability of the substance/molecule to elicit a desired response in the individual. A therapeutically effective amount also encompasses an amount in which any toxic or detrimental consequences of the substance/molecule are outweighed by the therapeutically beneficial effects. The "prophylactically effective amount" refers to an amount effective at the necessary dose and time to achieve the desired prophylactic effect. Usually, but not necessarily, the prophylactically effective amount will be less than the therapeutically effective amount because the prophylactic dose is administered to the subject prior to the onset of the disease or at an early stage of the disease. In the case of cancer, the therapeutically effective amount of the drug reduces the number of cancer cells; shrinks the tumor size; inhibits (i.e., slows to some extent, preferably stops) infiltration of cancer cells into surrounding organs; inhibits (i.e., slows to some extent, preferably stops) tumor metastasis; inhibits tumor growth in some degree; and/or alleviates one or more symptoms associated with cancer in some degree.

In the present invention, the 20 conventional amino acids and their abbreviations follow conventional usage. See Immunology-A Synthesis (2nd Edition, E.S. Golub and D.R. Gren, Eds., Sinauer Associates, Sunderland, Mass. (1991)), incorporated herein by reference.

The term "chimeric antibody" refers to an antibody in which the variable region sequences are derived from one species and the constant region sequences are derived from another species, such as antibodies in which the variable region sequences are derived from a mouse antibody and the constant region sequences are derived from a human antibody.

"Humanized" antibodies refer to non-human (e.g., mouse) forms of antibodies that are chimeric immunoglobulins, immunoglobulin chains, or fragments thereof (e.g., Fv, Fab, Fab', F(ab')2 or other antigen-binding subsequences of antibodies) containing a minimal sequence derived from non-human immunoglobulins. Preferably, the humanized antibody is a human immunoglobulin (recipient antibody) in which the complementarity determining region (CDR) residues of the recipient antibody are derived from CDR residue substitutions of a non-human species with the desired specificity, affinity and capacity (donor antibody) such as mouse, rat or rabbit.

In addition, in humanization, it is also possible to mutate amino acid residues within the CDR1, CDR2 and/or CDR3 regions of VH and/or VL, thereby improving one or more binding properties (e.g., affinity) of the antibody. For example, PCR-mediated mutagenesis can be performed to introduce mutations whose effect on antibody binding or other functional properties can be assessed using the in vitro or in vivo assays described herein. Typically, conservative mutations are introduced. Such mutations can be amino acid substitutions, additions or deletions. In addition, there are usually no more than one or two mutations within a CDR. Accordingly, the humanized antibodies of the present invention also encompass antibodies comprising 1 or 2 two amino acid mutations within the CDR.

The present invention will be further elucidated below using specific examples, but these examples do not limit the scope of the present invention.

Example 1 Preparation of Humanized Antibody

1. Preparation of Anti-Claudin 18.2 Monoclonal Antibody

Multiple strategies were simultaneously applied to immunize Balb/c mice and screen to obtain monoclonal antibodies that specifically bind to only Claudin 18.2, but not Claudin 18.1, a different splice variant of Claudin 18, as follows:

1) Construction of stably transfected cell line expressing Claudin 18.2

The plasmid Claudin 18.1-puc57-Amp (SynbioTech) containing the full-length gene sequence of human Claudin 18.1 (UniProtKB-P56856) was synthesized. Using this plasmid as a template, the upstream primer 5'-ttggcaaagaattgctagatgtccaccaccacatgcc-3' (SEQ ID NO: 171), and the downstream primer 5'-tgttcgggccctcctcgattacacatagtcgtgcttgg-3' (SEQ ID NO: 172), the full length of human Claudin 18.1 fragment (Met1-Val261) was amplified by PCR. The amplified product was enzymatically ligated by NEBuilder HiFi DNA Assembly Master Mix (NEB, Cat: M0530L), and then cloned into an eukaryotic expression plasmid system. Similarly, a plasmid Claudin 18.2-puc57-Amp (SynbioTech) containing the full-length gene sequence of human Claudin 18.2 (UniProtKB-P56856-2) was synthesized. Using this plasmid as a template, the upstream primer 5'-ttggcaaagaattgctagatggccgtgactgcctgtc-3' (SEQ ID NO: 173), and the downstream primer 5'-tgttcgggccctcctcgattacacatagtcgtgcttgg-3' (SEQ ID NO: 174), the full length of human Claudin 18.2 fragment (Met1-Val261) was amplified by PCR. The amplified product was enzymatically ligated by NEBuilder HiFi DNA Assembly Master Mix (NEB, Cat. No.: M0530L), and then cloned into an eukaryotic expression plasmid system. NIH3T3 and

HEK293 cells were electroporated with this plasmid, respectively, and 1-10 μg/mL Puromycin (Gibco, Cat. No.: A1113803) was used for pressurized screening stepwise to obtain stable expression cell lines.

The obtained HEK293 stably transfected cell lines expressing Claudin 18.1 and HEK293stably transfected cell lines expressing Claudin 18.2 were first verified by RT-PCR method. Total RNA was extracted from positive cell clones with Trizol RNA extraction kit, and reverse transcription kit (SuperScriptTM First-Strand Synthesis System, Cat. No.: 18080051) was used to obtain cDNA library by reverse transcription with Oligo (dT) primers. Since the two splice fragments of Claudin, Claudin 18.1 and 18.2 differ in the region from the N-terminus to the first extracellular domain (Loop1), the primers KNB14 (5'-tgtgcgccaccatggccgtg-3' (SEQ ID NO: 175)) and KNB15 (5'-tggaaggataagattgtacc-3' (SEQ ID NO: 176)) were designed, which can amplify the region (504bp) from Loop1 to the C-terminus (not including Loop1) of Claudin 18.1 and Claudin 18.2; the primer KNB16 ( 5'-tgggtgccattggcctcctg-3' (SEQ ID NO: 177) was designed, which can specifically and complementarily bind to the N-terminus of Claudin 18.2, but not bind to the N-terminus of Claudin 18.1, and only the full-length fragment (780 bp) of Claudin 18.2 from N-terminus to the C-terminus was amplified, and KATO III cells (ATCC HTB-103) expressing Claudin 18.2 was used as a positive control. As shown in Fig. 1, RT-PCR showed that the HEK293 stably transfected cell lines expressed Claudin 18.1 and Claudin 18.2, respectively. Both HEK293 stably transfected cell line expressing Claudin 18.2 and control KATO III cells can amplify a characteristic band of 780bp specific for Claudin 18.2, while HEK293 expressing Claudin 18.1 can only amplify a shared fragment of 504bp.

The stably transfected cell lines HEK293 cells expressing Claudin 18.2 and NIH3T3 cells expressing Claudin 18.2 were digested and collected, washed twice with PBS, and incubated with 100 μL of 1:200 diluted primary antibody rabbit anti-Claudin 18.2 (Abcam, EPR19202, Cat: ab222512) at 4°C for 60 minutes. Excess primary antibody solution was washed off with 0.5% BSA/PBS, and 50 μL of secondary antibody, goat anti-rabbit IgGFc-AF647 (Jackson ImmunoResearch, Cat: 111-606-046), was added and incubated at 4°C for 45 minutes. Excess secondary antibody was washed off with 0.5% BSA/PBS, and cells were finally resuspended with 100 μL of PBS solution, and immediately detected by flow cytometry. The results were shown in Fig. 2 and Fig. 3.

Fig. 2 shows the results of HEK293-Claudin 18.2 stably transfected cell lines transfected with the full-length Claudin 18.2 gene detected by FACS. The black dots are untransfected HEK293 cells, and the gray dots are HEK293 stably transfected cells expressing Claudin 18.2. HEK293 cells do not express Claudin 18.2, and HEK293-Claudin 18.2 stably transfected cells highly express Claudin 18.2 on the cell surface.

Fig. 3 shows the results of Claudin 18.2 in NIH3T3 stably transfected cell lines expressing high levels of Claudin 18.2 detected by FACS. The black line is the negative control, and the gray shading is the 3T3 stably transfected cell line expressing Claudin 18.2. NO.32-H is the 3T3 stably transfected cell line expressing high levels of Claudin 18.2, NO.18-M is the 3T3 stably transfected cell line expressing medium levels of Claudin 18.2, and NO. 6-L is a 3T3 stably transfected cell line expressing low levels of Claudin 18.2.

The positive stably transfected cell lines that had been expanded were collected and frozen, and the NIH3T3 stably transfected cell line expressing Claudin 18.2 was used to immunize animals.

2) Preparation of anti-Claudin 18.2 monoclonal antibodies from hybridomas

6-8-week old female Balb/c mice were immunized with 3T3 stably transfected cells expressing claudin 18.2 or the plasmid encoding Claudin 18.2, respectively, or alternately. When immunized with cells, $1\times10^6$ 3T3 stably transfected cells expressing claudin 18.2 were mixed with Freund's adjuvant or non-Freund's adjuvant each time, then injected into the thigh root and footpad, and immunization was performed again at different sites two weeks later. When DNA was used for immunization, 20 μg of plasmid was mixed with 1 μg of CpG, and then directly injected into the abdomen of mice with a gene gun (Biorad) at 40 psi, and immunization was performed once a week. Three days before fusion, the HEK293 stably transfected cell line expressing high levels of Claudin 18.2 was used for tail vein injection with $1\times10^6$ cells/50 μL per mouse for pulse immunization. Three days later, the mice were sacrificed, and the popliteal, inguinal, and iliac lymph nodes were collected and ground in DMEM to obtain a B cell-rich suspension; the mouse spleen was removed, ground in DMEM, and centrifuged to obtain a spleen cell suspension. An appropriate amount of mixture of lymph node and spleen cell suspension was mixed with SP2/0, and the cells were fused using an electrofusion apparatus.

3) Construction of anti-Claudin 18.2 phage antibody library

Part of the collected mouse spleen and peripheral lymph node cell suspension was used to extract total RNA with Trizol RNA extraction kit, and reverse transcription was carried out using the reverse transcription kit (SuperScript First-Strand Synthesis System, Cat. No. 18080051) with the light- and heavy chain specific primers to generate the antibody light- and heavy chain cDNA libraries, respectively. Using this cDNA library as a template, the light- and heavy chain variable region primers were used to amplify the antibody light- and heavy chain variable region fragments by PCR, and after enzyme digestion the PCR products were cloned into a phage plasmid vector containing human antibody light chain constant region Ckappa or human IgG1 heavy chain constant region CH1, which form chimeric light chain

library and heavy chain library, respectively. The antibody fragments in the light chain library were double digested with BspQI and SfiI, and then ligated into the heavy chain library to form a mouse chimeric Fab phage display library based on filamentous phage M13, with a library capacity of $1.2\times10^{10}$. 0.8 mL of phage (titer about $1\times10^{13}$/mL) was taken and mixed with 200 μL of 5% BSA/PBS, added with $1\times10^7$ HEK293 cells expressing claudin 18.1, and placed in ice bath for 1 hour; after centrifugation at 1000 rpm for 10 minutes, the collected supernatant was mixed with $1\times10^6$ HEK293 cells expressing Claudin 18.2, ice bathed for 1 hour, centrifuged at 1000 rpm for 3 minutes, and the supernatant was discarded; 1 mL of 1% BSA/PBS was added, and washed repeatedly for 5-10 times; 1 mL of 100 mM TEA was added to lyse cells, 0.5 mL of 1M Tris-HCl, pH7.5 was added for neutralization after incubation for 10 minutes at room temperature, and 10 mL of TG1 *E. coli.* in logarithmic growth phase was used for phage infection at 37°C for 30 minutes. According to the routine procedures of molecular biology, the phages were recovered, the titer was detected and the next round of panning was performed.

2. Screening and sequence acquisition of anti-Claudin 18.2-specific antibodies

HEK293 expressing Claudin 18.1, HEK293 expressing Claudin 18.2, and HEK293 cells were pre-stained with 5 μM, 0.5 μM, and 0 μM Cell Tracker Green CMFDA Dye (Thermo, Cat. No.: C2925), respectively, according to the instructions for live cell staining. After washing off the dye, cells were mixed at a ratio of 1:1:1, added to 96-well plates ($2\times10^5$ cells/well), combined with hybridoma supernatant or supernatant obtained by bacterial induction and incubated in ice bath for 1 hour. AlexaFluro647-labeled secondary antibody anti-mouse IgG Fc or anti-human IgG F(ab)'2 (Jackson ImmunoResearch) was added, and incubated on ice for 45 minutes. After washing, 100 μL of PBS was added to each well to resuspend the cells, the cells were analyzed by the flow cytometer (iQue Screener), three different cell populations were circled according to the difference in the fluorescence intensity of the FL2 channel, and then the binding of the antibody to be tested to each cell population in the FL4 channel was detected. The screened antibody binds to HEK293 stably transfected cells expressing Claudin 18.2 with high affinity and specificity, and does not bind to HEK293 stably transfected cells expressing Claudin 18.1 or HEK293 cells. From the hybridoma cells, a total of 320 clones were screened by FACS and said 320 clones could bind to Claudin 18.2, but not to Claudin 18.1; after 3 rounds of panning, 62 clones were selected from the phage Fab library and said 62 clones bind to Claudin 18.2 with high affinity but not to Claudin 18.1.

The plasmids were extracted from the positive clones screened from the phage library for sequencing, and the variable region sequences were cloned into the heavy chain and light chain constant region vectors, respectively, for full-length IgG expression. The positive cells obtained from the hybridoma were lysed by adding 1 mL of TRNzol, and the total RNA was extracted by the guanidine thiocyanate method. Using this as a template, after synthesizing the first-strand cDNA, the first-strand cDNA was used as a subsequent template to amplify the DNA sequence of the variable region corresponding to the hybridoma cells. After sequencing the amplified products, the variable region sequences of the heavy and light chains of the candidate hybridomas were obtained, as shown below.

Clone 18D10:

Heavy Chain

**DVQLQESGPGLVKPSQSLSLTCTVT <u>GYSITSNYAWN</u> WIRQFPGNKLEWMG <u>YINYSGNTNYNPSLKS</u> RISITRDTSKNQFFLQLNSVTAEDTATYYCAT <u>SYYGNSFIY</u> WGQGTLVTVSA (SEQ ID NO: 139).**

Therein the underlined parts are CDR1 (SEQ ID NO: 140), CDR2 (SEQ ID NO: 141), CDR3 (SEQ ID NO: 142) from left to right, respectively; and

The non-underlined parts are FR1 (SEQ ID NO: 143), FR2 (SEQ ID NO: 144), FR3 (SEQ ID NO: 145), and FR4 (SEQ ID NO: 146) from left to right, respectively.

Nucleic acid sequence

GATGTGCAGCTTCAGGAGTCGGGACCTGGCCTGGTGAAACCTTCTCAGTCTCTGTCCCTCACCTGCACTGTCACTGGCTACTCAATCACCAGTAATTATGCCTGGAACTGGATCCGACAGTTTCCAGGAAACAAACTAGAGTGGATGGGCTACATAAACTACAGTGGGAACACTAACTATAACCCATCTCTCAAAAGTCGAATCTCTATCACTCGAGACACATCCAAGAACCAGTTCTTCCTGCAGTTGAATTCTGTGACTGCTGAGGACACAGCCACATATTATTGTGCAACCTCCTATTATGGTAATTCCTTTATTTACTGGGGCCAAGGGACTCTGGTCACTGTCTCTGCA (SEQ ID NO: 178).

Light chain

DIVMTQSPSSLTVTAGEKVTMSCKSSQSLLNSGNQKNYLTWYQQKPGQPPKLLIYWASTRESGVPDRFTGSGSGTDFTLTISSVQAEDLAVYYCQNAYSFPWTFGGGTKLEIK (SEQ ID NO: 147).

Therein the underlined parts are CDR1 (SEQ ID NO: 148), CDR2 (SEQ ID NO: 149), CDR3 (SEQ ID NO: 150) from left to right, respectively; and

The non-underlined parts are FR1 (SEQ ID NO: 151), FR2 (SEQ ID NO: 152), FR3 (SEQ ID NO: 153), and FR4 (SEQ ID NO: 154) from left to right, respectively.

Nucleic acid sequence

GACATTGTGATGACACAGTCTCCATCCTCCCTGACTGTGACAGCAGGAGAGAAGGTCACTATGAGCTGCAAGTCCAGTCAGAGTCTGTTAAACAGTGGAAATCAAAAGAACTACTTGACCTGGTACCAGCAGAAACCAGGGCAGCCTCCTAAACTGTTGATCTACTGGGCATCCACTAGGGAGTCTGGGGTCCCTGATCGCTTCACAGGCAGTGGATCTGGAACAGATTTCACTCTCACCATCAGCAGTGTGCAGGCTGAAGACCTGGCAGTTTATTACTGTCAGAATGCTTATAGTTTTCCGTGGACGTTCGGTGGAGGCACCAAGCTGGAAATCAAA (SEQ ID NO: 179).

Clone 18A9:

Heavy Chain

QVQLQQSGREVVRPGTSVKVSCKPSGYAFTNYLIDWVKQRPGQGLEWIGGINPGSGDTVYNEKFKAKATLTADKSSMTANMQLSSLTSDDSAVYFCARRVRGNSFDSWGQGTLVTVSA (SEQ ID NO: 155).

Therein the underlined parts are CDR1 (SEQ ID NO: 156), CDR2 (SEQ ID NO: 157), CDR3 (SEQ ID NO: 158) from left to right, respectively; and

The non-underlined parts are FR1 (SEQ ID NO: 159), FR2 (SEQ ID NO: 160), FR3 (SEQ ID NO: 161), FR4 (SEQ ID NO: 162) from left to right, respectively.

Nucleic acid sequence

CAGGTGCAGCTGCAGCAGTCTGGACGTGAGGTGGTAAGGCCTGGGACTTCAGTGAAGGTGTCCTGCAAGCCTTCTGGATACGCCTTCACTAATTACTTGATAGACTGGGTAAAACAGAGGCCTGGACAGGGCCTTGAGTGGATTGGAGGGATTAATCCTGGAAGTGGTGACACTGTGTACAATGAGAAGTTCAAGGCCAAGGCAACACTGACTGCAGACAAATCCTCCATGACTGCCAACATGCAGCTCAGCAGCCTGACATCTGATGACTCTGCGGTCTATTTCTGCGCAAGAAGGGTCCGTGGTAATTCGTTTGATTCCTGGGGCCAAGGGACTCTGGTCACTGTCTCTGCA (SEQ ID NO: 180).

Light chain

DIVMSQSPSSLTVTAGEKVTMSCKSSQSLLNSGNQKNYLTWYQQKPGQPPKLLIYWASTRESGVPDRFTGSGSGKDFTLTISSVQAEDLALYYCQNNYFYPLTFGAGTKLELK (SEQ ID NO: 163).

Therein the underlined parts are CDR1 (SEQ ID NO: 164), CDR2 (SEQ ID NO: 165), CDR3 (SEQ ID NO: 166) from left to right, respectively; and

The non-underlined parts are FR1 (SEQ ID NO: 167), FR2 (SEQ ID NO: 168), FR3 (SEQ ID NO: 169), and FR4 (SEQ ID NO: 170) from left to right, respectively.

Nucleic acid sequence

GACATTGTGATGTCACAGTCTCCATCCTCCCTGACTGTGACAGCAGGAGAGAAGGTCACTATGAGCTGCAAGTCCAGTCAGAGTCTGTTAAACAGTGGAAATCAAAAGAACTACTTGACCTGGTACCAGCAGAAACCAGGGCAGCCTCCTAAATTGTTGATCTACTGGGCATCCACTAGGGAATCTGGGGTCCCTGATCGCTTCACAGGCAGTGGATCTGGAAAAGATTTCACTCTCACCATCAGCAGTGTGCAGGCTGAAGACCTGGCACTTTATTACTGTCAGAATAATTATTTTTATCCGCTCACGTTCGGTGCTGGGACCAAGCTGGAGCTGAAA (SEQ ID NO: 181).

The above heavy and light chain variable region sequence fragments were amplified by PCR, and the heavy chain variable region was cloned into a vector containing the human heavy chain constant region to express the intact IgG1 heavy chain in mammalian cells. Similarly, the light chain variable region was cloned into a vector containing the human light chain constant region to express the complete kappa light chain in mammalian cells. After verifying the sequences, they were transfected into HEK293-6E mammalian cells, IgG1 was expressed and secreted into the medium, and the supernatant was collected, filtered and then purified. The IgG was purified by Protein A chromatography, and the culture supernatant was loaded on an appropriately sized Protein A column, washed with 50 mM Tris-HCl pH 8.0, 250 mM NaCl, and the bound IgG was eluted with 0.1 M Glycine-HCl, pH 3.0. The protein was concentrated by ultrafiltration using a concentration tube (Millipore), and the concentration of IgG was determined by spectrophotometry through detecting OD280. The purity of IgG was analyzed by SDS-PAGE.

HEK293 expressing Claudin 18.1, HEK293 expressing Claudin 18.2 and HEK293 cells were harvested in logarithmic growth phase, after they were digested, $5 \times 10^4$ cells/100 μL were added to a U-shaped 96-well plate, centrifuged at 1100 rpm for 3 minutes, then the supernatant was discarded. The cells were gently tapped, and 50 μL of serially diluted antibodies were added to each well (antibody concentration starting from 100 nM, 5-fold dilution for 8 gradients), and incubated at 4°C for 1 hour. After the incubation, 140 μL of 0.5% BSA was added to each well to wash 3 times, and 30 μL/well of secondary antibody AlexaFluro647 anti-human IgG (Jackson ImmunoResearch, Cat: 109-606-170) was added, and incubated at 4°C for 40 minutes. After the incubation, 140 μL of 0.5% BSA was added to each well to wash 3 times, and finally each well was resuspended in 50 μL of PBS for detection by flow cytometry (iQue Screener). As shown in Table 1, the results show that the obtained chimeric Claudin 18.2 IgG1 antibody recognizes only HEK293 cells transfected with Claudin 18.2 gene expressing Claudin 18.2, but has no binding to HEK293 or HEK293 expressing Claudin 18.1.

Table 1: Binding of Claudin 18.2 antibodies to stably transfected cell lines (N.B. indicates no binding detected)

| mAb | Claudin 18.2 | Claudin 18.1 | HEK293 |
|---|---|---|---|
| | $EC_{50}$ (nM) | $EC_{50}$ (nM) | $EC_{50}$ (nM) |
| Clone 18D10 | 0.37 | N.B. | N.B. |
| Clone 18A9 | 0.31 | N.B. | N.B. |

3. Humanization of Anti-Claudin 18.2 Antibody

The selected monoclonal antibody variable region sequences were aligned with the human germline antibody sequences to find out the sequence with high homology for CDR grafting; subsequently, homology modeling was performed in silico to analyze the CDR region and its surrounding framework amino acid sequence to investigate its spatial three-dimensional binding mode. By calculating the electrostatic force, van der Waals force, hydrophobicity and entropy value, the key amino acid residues, which may interact with the target and maintain the spatial framework, in each positive monoclonal antibody gene sequence are analyzed, and the back mutation sites are designed accordingly. The HLA-DR affinity was analyzed and the human germline framework sequences with less immunogenicity were selected. Amino acid residues that may be modified during fermentation were analyze and mutations were designed to reduce the likelihood of modification.

Different heavy chain and light chain derivatives were designed. After full sequence synthesis of the light- and heavy chain derivatives, they were cloned into a vector containing the constant region Ckappa of the antibody kappa chain or the constant region CH1-CH3 of human IgG1. After the plasmids harboring the same parent-derived light- and heavy chain derivatives respectively were paired, HEK293.6E cells were transfected to express antibodies for 5-6 days, and the supernatant was collected and purified on a Protein A column.

The sequences of the humanized antibodies are as follows: 18D10:

Heavy chain variable region:
18D10VHv1:

**QVQLQESGPGLVKPSETLSLTCTVS <u>GYSITSNYAWN</u> WIRQPPGKGLEWIG <u>YINYSGNTNYNPSLKS</u> RVTISRDTSKNQFSLKLSSVTAADTAVYYCAT <u>SYYGNSFIY</u> WGQGTLVTVSS (SEQ ID NO: 1).**

Therein the underlined parts are CDR1 (SEQ ID NO:2), CDR2 (SEQ ID NO:3), CDR3 (SEQ ID NO:4) from left to right, respectively; and

The non-underlined parts are FR1 (SEQ ID NO: 5), FR2 (SEQ ID NO: 6), FR3 (SEQ ID NO: 7), and FR4 (SEQ ID NO: 8) from left to right, respectively.

Nucleic acid sequence

**CAGGTTCAGCTGCAAGAGTCTGGACCTGGCCTGGTCAAGCCTAGCGAGACACTGAGCCTGACCTGTACCGTGTCCGGCTACAGCATCACCAGCAACTACGCCTGGAACTGGATCAGACAGCCTCCTGGCAAAGGCCTCGAGTGGATCGGCTACATCAACTACAGCGGCAACACCAACTACAACCCCAGCCTGAAGTCCAGAGTGACCATCAGCAGAGACACCAGCAAGAACCAGTTCTCCCTGAAGCTGAGCAGCGTGACAGCCGCCGATACAGCCGTGTACTACTGTGCCACAAGCTACTACGGCAACAGCTTCATCTACTGGGGCCAGGGCACACTGGTCACCGTTTCTTCT (SEQ ID NO: 182).**

18D10VHv2:

**QVQLQESGPGLVKPSETLSLTCTVS <u>GGSISSNYAWN</u> WIRQPPGKGLEWMG <u>YINYSGNTNYNPSLKS</u> RITISRDTSKNQFSLKLSSVTAADTAVYYCAT <u>SYYGNSFIY</u> WGQGTLVTVSS (SEQ ID NO: 9).**

Therein the underlined parts are CDR1 (SEQ ID NO: 10), CDR2 (SEQ ID NO: 11), CDR3 (SEQ ID NO: 12) from left to right, respectively;

The non-underlined parts are FR1 (SEQ ID NO: 13), FR2 (SEQ ID NO: 14), FR3 (SEQ ID NO: 15), and FR4 (SEQ ID NO: 16) from left to right, respectively.

Nucleic acid sequence

**CAGGTTCAGCTGCAAGAGTCTGGACCTGGCCTGGTCAAGCCTAGCGAGACACTGAGCCTGACCTGTACCGTGTCCGGCGGCAGCATCAGCAGCAACTACGCCTGGAACTGGATCAGACAGCCTCCTGGCAAAGGCCTCGAGTGGATGGGCTACATCAACTACAGCGGCAACACCAACTACAACCCCAGCCTGAAGTCCAGAATCACCATCAGCAGAGACACCAGCAAGAACCAGTTCTCCCTGAAGCTGAGCAGCGTGACAGCCGCCGATACAGCCGTGTACTACTGTGCCACAAGCTACTACGGCAACAGCTTCATCTACTGGGGCCAGGGCACACTGGTCACCGTTTCTTCT (SEQ ID NO: 183).**

18D10VHv3:

**QVQLQESGPGLVKPSETLSLTCTVSGGSISSNYAWNWIRQPPGKGLEWIGYINYSGNTNYNPSLKSRVTISRDTSKNQFSLKLSSVTAADTAVYYCATSYYGNSFIYWGQGTLVTVSS (SEQ ID NO: 17).**

Therein the underlined parts are CDR1 (SEQ ID NO: 18), CDR2 (SEQ ID NO: 19), CDR3 (SEQ ID NO: 20) from left to right, respectively; and

The non-underlined parts are FR1 (SEQ ID NO: 21), FR2 (SEQ ID NO: 22), FR3 (SEQ ID NO: 23), and FR4 (SEQ ID NO: 24) from left to right, respectively.

Nucleic acid sequence

**CAGGTTCAGCTGCAAGAGTCTGGACCTGGCCTGGTCAAGCCTAGCGAGACACTGAGCCTGACCTGTACCGTGTCCGGCGGCAGCATCAGCAGCAACTACGCCTGGAACTGGATCAGACAGCCTCCTGGCAAAGGCCTCGAGTGGATCGGCTACATCAACTACAGCGGCAACACCAACTACAACCCCAGCCTGAAGTCCAGAGTGACCATCAGCAGAGACACCAGCAAGAACCAGTTCTCCCTGAAGCTGAGCAGCGTGACAGCCGCCGATACAGCCGTGTACTACTGTGCCACAAGCTACTACGGCAACAGCTTCATCTACTGGGGCCAGGGCACACTGGTCACCGTTTCTTCT (SEQ ID NO: 184).**

18D10VHv4:

**QVQLQESGPGLVKPSETLSLTCTVS GGSISSNYAWN WIRQPPGKGLEWIG YINYSGYTNYNPSLKS RVTISRDTSKNQFSLKLSSVTAADTAVYYCAT SYYGNSFIY WGQGTLVTVSS (SEQ ID NO: 25).**

Therein the underlined parts are CDR1 (SEQ ID NO: 26), CDR2 (SEQ ID NO: 27), CDR3 (SEQ ID NO: 28) from left to right, respectively; and

The non-underlined parts are FR1 (SEQ ID NO: 29), FR2 (SEQ ID NO: 30), FR3 (SEQ ID NO: 31), and FR4 (SEQ ID NO: 32) from left to right, respectively.

Nucleic acid sequence

CAGGTTCAGCTGCAAGAGTCTGGACCTGGCCTGGTCAAGCCTAGCGAGAC ACTGAGCCTGACCTGTACCGTGTCCGGCGGCAGCATCAGCAGCAACTACGCCT GGAACTGGATCAGACAGCCTCCTGGCAAAGGCCTCGAGTGGATCGGCTACATC AACTACAGCGGCTACACCAACTACAACCCCAGCCTGAAGTCCAGAGTGACCATC AGCAGAGACACCAGCAAGAACCAGTTCTCCCTGAAGCTGAGCAGCGTGACAGC CGCCGATACAGCCGTGTACTACTGTGCCACAAGCTACTACGGCAACAGCTTCAT CTACTGGGGCCAGGGCACACTGGTCACCGTTTCTTCT (SEQ ID NO: 185).

18D10VHv5:

QVQLQESGPGLVKPSETLSLTCTVS GGSISSNYAWN WIRQPPGKGLEWIG YINYSGNTAYNPSLKS RVTISRDTSKNQFSLKLSSVTAADTAVYYCAT SYYGNSFIY

WGQGTLVTVSS (SEQ ID NO: 33).

Therein the underlined parts are CDR1 (SEQ ID NO:34), CDR2 (SEQ ID NO:35), CDR3 (SEQ ID NO:36) from left to right, respectively; and

The non-underlined parts are FR1 (SEQ ID NO: 37), FR2 (SEQ ID NO: 38), FR3 (SEQ ID NO: 39), and FR4 (SEQ ID NO: 40) from left to right, respectively.

Nucleic acid sequence

CAGGTTCAGCTGCAAGAGTCTGGACCTGGCCTGGTCAAGCCTAGCGAGAC ACTGAGCCTGACCTGTACCGTGTCCGGCGGCAGCATCAGCAGCAACTACGCCT GGAACTGGATCAGACAGCCTCCTGGCAAAGGCCTCGAGTGGATCGGCTACATC AACTACAGCGGCAACACCGCCTACAACCCCAGCCTGAAGTCCAGAGTGACCAT CAGCAGAGACACCAGCAAGAACCAGTTCTCCCTGAAGCTGAGCAGCGTGACAG CCGCCGATACAGCCGTGTACTACTGTGCCACAAGCTACTACGGCAACAGCTTCA TCTACTGGGGCCAGGGCACACTGGTCACCGTTTCTTCT (SEQ ID NO: 186).

18D10VHv6:

QVQLQESGPGLVKPSETLSLTCTVS GGSISSNYAWN WIRQPPGKGLEWIG YIYYSGNTNYNPSLKS RVTISRDTSKNQFSLKLSSVTAADTAVYYCAT SYYGNSFIY WGQGTLVTVSS (SEQ ID NO: 41).

Therein the underlined parts are CDR1 (SEQ ID NO: 42), CDR2 (SEQ ID NO: 43), CDR3 (SEQ ID NO: 44) from left to right, respectively; and

The non-underlined parts are FR1 (SEQ ID NO: 45), FR2 (SEQ ID NO: 46), FR3 (SEQ ID NO: 47), FR4 (SEQ ID NO: 48) from left to right, respectively.

Nucleic acid sequence

CAGGTTCAGCTGCAAGAGTCTGGACCTGGCCTGGTCAAGCCTAGCGAGAC ACTGAGCCTGACCTGTACCGTGTCCGGCGGCAGCATCAGCAGCAACTACGCCT GGAACTGGATCAGACAGCCTCCTGGCAAAGGCCTCGAGTGGATCGGCTACATC TACTACAGCGGCAACACCAACTACAACCCCAGCCTGAAGTCCAGAGTGACCATC AGCAGAGACACCAGCAAGAACCAGTTCTCCCTGAAGCTGAGCAGCGTGACAGC CGCCGATACAGCCGTGTACTACTGTGCCACAAGCTACTACGGCAACAGCTTCAT CTACTGGGGCCAGGGCACACTGGTCACCGTTTCTTCT (SEQ ID NO: 187).

Light chain variable region:
18D10VLv1:

DIVMTQSPDSLAVSLGERATINC KSSQSLLNSGNQKNYLTWYQQKPGQPPKLLIY WASTRES GVPDRFSGSGSGTDFTLTISSLQAEDVAVYYC QNAYSFPWT FGQGTKVEIK (SEQ ID NO: 49).

Therein the underlined parts are CDR1 (SEQ ID NO: 50), CDR2 (SEQ ID NO: 51), CDR3 (SEQ ID NO: 52) from left to right, respectively; and

The non-underlined parts are FR1 (SEQ ID NO: 53), FR2 (SEQ ID NO: 54), FR3 (SEQ ID NO: 55), and FR4 (SEQ ID NO: 56) from left to right, respectively.

Nucleic acid sequence

GACATCGTGATGACACAGAGCCCTGATAGCCTGGCCGTGTCTCTGGGAGA GAGAGCCACCATCAACTGCAAGAGCAGCCAGAGCCTGCTGAACAGCGGCAACC AGAAGAACTACCTGACCTGGTATCAGCAGAAGCCCGGCCAGCCTCCTAAGCTG CTGATCTACTGGGCCAGCACCAGAGAAAGCGGCGTGCCAGATAGATTCAGCGG CAGCGGCTCTGGAACCGACTTCACCCTGACAATCAGCTCCCTGCAGGCCGAGG ATGTGGCCGTGTACTACTGTCAGAACGCCTACAGCTTCCCCTGGACATTCGGCC AGGGCACCAAGGTGGAAATCAAG (SEQ ID NO: 188).

18D10VLv2:

DIVMTQSPDSLAVSLGERATINC KSSQSLLNSGNQKNYLA WYQQKPGQPPKLLIY WASTRES GVPDRFSGSGSGTDFTLTISSLQAEDVAVYYC QQAYSFPWT FGQGTKVEIK (SEQ ID NO: 57).

Therein the underlined parts are CDR1 (SEQ ID NO: 58), CDR2 (SEQ ID NO: 59), CDR3 (SEQ ID NO: 60) from left to right, respectively; and

The non-underlined parts are FR1 (SEQ ID NO: 61), FR2 (SEQ ID NO: 62), FR3 (SEQ ID NO: 63), and FR4 (SEQ ID NO: 64) from left to right, respectively.

Nucleic acid sequence

GACATCGTGATGACACAGAGCCCTGATAGCCTGGCCGTGTCTCTGGGAGA GAGAGCCACCATCAACTGCAAGAGCAGCCAGAGCCTGCTGAACAGCGGCAACC AGAAGAACTACCTGGCCTGGTATCAGCAGAAGCCCGGCCAGCCTCCTAAGCTG CTGATCTACTGGGCCAGCACCAGAGAAAGCGGCGTGCCAGATAGATTCAGCGG CAGCGGCTCTGGAACCGACTTCACCCTGACAATCAGCTCCCTGCAGGCCGAGG ATGTGGCCGTGTACTACTGTCAGCAGGCCTACAGCTTCCCCTGGACATTCGGCC AGGGCACCAAGGTGGAAATCAAG (SEQ ID NO: 189).

The sequences of preferred humanized antibodies are as follows:

Heavy Chain Amino Acid Sequence:

QVQLQESGPGLVKPSETLSLTCTVSGGSISSNYAWNWIRQPPGKGLEWIGYIYY SGNTNYNPSLKSRVTISRDTSKNQFSLKLSSVTAADTAVYYCATSYYGNSFIYWGQG TLVTVSSASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKRVEPKSCDKTH TCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTIS KAKGQPREPQVYTLPPSREEMTKNQVSLTCLVKGFYPSDIAVEWESNGQPENNYKT TPPVLDSDGSFFLYSKLTVDKSRWQQGNVFSCSVMHEALHNHYTQKSLSLSPGK (SEQ ID NO: 65).

Heavy chain nucleic acid sequence

CAGGTTCAGCTGCAAGAGTCTGGACCTGGCCTGGTCAAGCCTAGCGAGAC ACTGAGCCTGACCTGTACCGTGTCCGGCGGCAGCATCAGCAGCAACTACGCCT GGAACTGGATCAGACAGCCTCCTGGCAAAGGCCTCGAGTGGATCGGCTACATC TACTACAGCGGCAACACCAACTACAACCCCAGCCTGAAGTCCAGAGTGACCATC AGCAGAGACACCAGCAAGAACCAGTTCTCCCTGAAGCTGAGCAGCGTGACAGC CGCCGATACAGCCGTGTACTACTGTGCCACAAGCTACTACGGCAACAGCTTCAT CTACTGGGGCCAGGGCACACTGGTCACCGTTTCTTCTGCTAGCACCAAGGGCC CATCCGTCTTCCCCCTGGCACCCTCCTCCAAGAGCACCTCTGGGGGCACAGCG GCCCTGGGCTGCCTGGTCAAGGACTACTTCCCCGAACCGGTGACGGTGTCGTG GAACTCAGGCGCCCTGACCAGCGGCGTGCACACCTTCCCGGCTGTCCTACAGT CCTCAGGACTCTACTCCCTCAGCAGCGTGGTGACCGTGCCCTCCAGCAGCTTGG GCACCCAGACCTACATCTGCAACGTGAATCACAAGCCCAGCAACACCAAGGTG GACAAGAGAGTTGAGCCCAAATCTTGTGACAAAACTCACACATGCCCACCGTGC CCAGCACCTGAACTCCTGGGGGGACCGTCAGTCTTCCTCTTCCCCCCAAAACCC AAGGACACCCTCATGATCTCCCGGACCCCTGAGGTCACATGCGTGGTGGTGGA CGTGAGCCACGAAGACCCTGAGGTCAAGTTCAACTGGTACGTGGACGGCGTGG AGGTGCATAATGCCAAGACAAAGCCGCGGGAGGAGCAGTACAACAGCACGTAC CGTGTGGTCAGCGTCCTCACCGTCCTGCACCAGGACTGGCTGAATGGCAAGGA GTACAAGTGCAAGGTCTCCAACAAAGCCCTCCCAGCCCCCATCGAGAAAACCAT CTCCAAAGCCAAAGGGCAGCCCCGAGAACCACAGGTGTACACCCTGCCCCCAT CCCGGGAGGAGATGACCAAGAACCAGGTCAGCCTGACCTGCCTGGTCAAAGGC TTCTATCCCAGCGACATCGCCGTGGAGTGGGAGAGCAATGGGCAGCCGGAGAA CAACTACAAGACCACGCCTCCCGTGCTGGACTCCGACGGCTCCTTCTTCCTCTA TAGCAAGCTCACCGTGGACAAGAGCAGGTGGCAGCAGGGGAACGTCTTCTCAT GCTCCGTGATGCATGAGGCTCTGCACAACCACTACACGCAGAAGTCCCTCTCCC

TGTCTCCGGGTAAATGA (SEQ ID NO: 190).

Light Chain Amino Acid Sequence:

DIVMTQSPDSLAVSLGERATINCKSSQSLLNSGNQKNYLTWYQQKPGQPPKLLIYWASTRESGVPDRFSGSGSGTDFTLTISSLQAEDVAVYYCQNAYSFPWTFGQGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO: 66).

Light chain nucleic acid sequence

GACATCGTGATGACACAGAGCCCTGATAGCCTGGCCGTGTCTCTGGGAGAGAGAGCCACCATCAACTGCAAGAGCAGCCAGAGCCTGCTGAACAGCGGCAACCAGAAGAACTACCTGACCTGGTATCAGCAGAAGCCCGGCCAGCCTCCTAAGCTGCTGATCTACTGGGCCAGCACCAGAGAAAGCGGCGTGCCAGATAGATTCAGCGGCAGCGGCTCTGGAACCGACTTCACCCTGACAATCAGCTCCCTGCAGGCCGAGGATGTGGCCGTGTACTACTGTCAGAACGCCTACAGCTTCCCCTGGACATTCGGCCAGGGCACCAAGGTGGAAATCAAGCGAACTGTGGCTGCACCATCTGTCTTCATCTTCCCGCCATCTGATGAGCAGTTGAAATCTGGAACTGCCTCTGTTGTGTGCCTGCTGAATAACTTCTATCCCAGAGAGGCCAAAGTACAGTGGAAGGTGGATAACGCCCTCCAATCGGGTAACTCCCAGGAGAGTGTCACAGAGCAGGACAGCAAGGACAGCACCTACAGCCTCAGCAGCACCCTGACGCTGAGCAAAGCAGACTACGAGAAACACAAAGTCTACGCCTGCGAAGTCACCCATCAGGGCCTGAGCTCGCCCGTCACAAAGAGCTTCAACAGGGGAGAGTGTTAG (SEQ ID NO: 191).

18A9:
Heavy chain variable region:
18A9VHv1:

QVQLVQSGAEVKKPGSSVKVSCKPS <u>GYAFTNYLID</u> WVRQAPGQGLEWMG <u>GINPGSGDTVYNEKFQ</u> GRVTLTADKSSMTAYMELSSLRSEDTAVYFCAR <u>RVRGNSFDS</u> WGQGTLVTVSS (SEQ ID NO: 67).

Therein the underlined parts are CDR1 (SEQ ID NO: 68), CDR2 (SEQ ID NO: 69), CDR3 (SEQ ID NO: 70) from left to right, respectively; and

The non-underlined parts are FR1 (SEQ ID NO: 71), FR2 (SEQ ID NO: 72), FR3 (SEQ ID NO: 73), FR4 (SEQ ID NO: 74) from left to right, respectively.

Nucleic acid sequence

CAGGTTCAGCTGGTTCAGTCTGGCGCCGAAGTGAAGAAACCTGGCAGCAGCGTGAAGGTGTCCTGCAAGCCTTCTGGCTACGCCTTCACCAACTACCTGATCGACTGGGTCCGACAGGCTCCTGGACAGGGACTTGAATGGATGGGCGGCATCAACCCTGGCAGCGGCGATACAGTGTACAACGAGAAGTTCCAGGGCAGAGTGACCCTGACCGCCGACAAGTCTAGCATGACCGCCTACATGGAACTGAGCAGCCTGAGAAGCGAGGATACCGCCGTGTACTTCTGTGCCAGAAGAGTGCGGGGCAACAGCTTCGATTCTTGGGGCCAGGGAACCCTGGTCACCGTTTCTTCT(SEQ ID NO: 192).

18A9VHv2:

**QVQLVQSGAEVKKPGSSVKVSCKPSGYTFTNYLIDWVRQAPGQGLEWMGGINPGSGDTVYNEKFQGRVTLTADESTSTAYMELSSLRSEDTAVYFCARRVRGNSFDSWGQGTLVTVSS (SEQ ID NO: 75).**

Therein the underlined parts are CDR1 (SEQ ID NO: 76), CDR2 (SEQ ID NO: 77), CDR3 (SEQ ID NO: 78) from left to right, respectively; and

The non-underlined parts are FR1 (SEQ ID NO: 79), FR2 (SEQ ID NO: 80), FR3 (SEQ ID NO: 81), and FR4 (SEQ ID NO: 82) from left to right, respectively.

Nucleic acid sequence

**CAGGTTCAGCTGGTTCAGTCTGGCGCCGAAGTGAAGAAACCTGGCAGCAG
CGTGAAGGTGTCCTGCAAGCCTTCTGGCTACACCTTCACCAACTACCTGATCGA
CTGGGTCCGACAGGCTCCTGGACAGGGACTTGAATGGATGGGCGGCATCAACC
CTGGCAGCGGCGATACAGTGTACAACGAGAAGTTCCAGGGCAGAGTGACCCTG
ACCGCCGACGAGTCTACCAGCACCGCCTACATGGAACTGAGCAGCCTGAGAAG
CGAGGATACCGCCGTGTACTTCTGTGCCAGAAGAGTGCGGGGCAACAGCTTCG
ATTCTTGGGGCCAGGGAACCCTGGTCACCGTTTCTTCT (SEQ ID NO: 193).**

18A9VHv3:

**QVQLVQSGAEVKKPGSSVKVSCKPS GYTFTNYLID WVRQAPGQGLEWMG GINPGSGDTVYNEKFQGRVTLTADKSSMTAYMELSSLRSEDTAVYYCARRVRGNSFDS WGQGTLVTVSS (SEQ ID NO: 83).**

Therein the underlined parts are CDR1 (SEQ ID NO: 84), CDR2 (SEQ ID NO: 85), CDR3 (SEQ ID NO: 86) from left to right, respectively; and

The non-underlined parts are FR1 (SEQ ID NO: 87), FR2 (SEQ ID NO: 88), FR3 (SEQ ID NO: 89), and FR4 (SEQ ID NO: 90) from left to right, respectively.

Nucleic acid sequence

**CAGGTTCAGCTGGTTCAGTCTGGCGCCGAAGTGAAGAAACCTGGCAGCAG
CGTGAAGGTGTCCTGCAAGCCTTCTGGCTACACCTTCACCAACTACCTGATCGA
CTGGGTCCGACAGGCTCCTGGACAGGGACTTGAATGGATGGGCGGCATCAACC
CTGGCAGCGGCGATACAGTGTACAACGAGAAGTTCCAGGGCAGAGTGACCCTG
ACCGCCGACAAGTCTAGCATGACCGCCTACATGGAACTGAGCAGCCTGAGAAG
CGAGGATACCGCCGTGTACTACTGTGCCAGAAGAGTGCGGGGCAACAGCTTCG
ATTCTTGGGGCCAGGGAACCCTGGTCACCGTTTCTTCT (SEQ ID NO: 194).**

18A9VHv4:

**QVQLVQSGAEVKKPGSSVKVSCKAS GYTFTNYLID WVRQAPGQGLEWMG GINPGSGDTVYNEKFQ GRVTLTADKSSMTAYMELSSLRSEDTAVYFCAR RVRGNSFDS WGQGTLVTVSS (SEQ ID NO: 91).**

Therein the underlined parts are CDR1 (SEQ ID NO: 92), CDR2 (SEQ ID NO: 93), CDR3 (SEQ ID NO: 94) from left to right, respectively; and

The non-underlined parts are FR1 (SEQ ID NO: 95), FR2 (SEQ ID NO: 96), FR3 (SEQ ID NO: 97), and FR4 (SEQ ID NO: 98) from left to right, respectively.

Nucleic acid sequence

**CAGGTTCAGCTGGTTCAGTCTGGCGCCGAAGTGAAGAAACCTGGCAGCAG CGTGAAGGTGTCCTGCAAGGCTTCTGGCTACACCTTCACCAACTACCTGATCGA CTGGGTCCGACAGGCTCCTGGACAGGGACTTGAATGGATGGGCGGCATCAACC CTGGCAGCGGCGATACAGTGTACAACGAGAAGTTCCAGGGCAGAGTGACCCTG ACCGCCGACAAGTCTAGCATGACCGCCTACATGGAACTGAGCAGCCTGAGAAG CGAGGATACCGCCGTGTACTTCTGTGCCAGAAGAGTGCGGGGCAACAGCTTCG ATTCTTGGGGCCAGGGAACCCTGGTCACCGTTTCTTCT (SEQ ID NO: 195).**

18A9VHv5:

**QVQLVQSGAEVKKPGSSVKVSCKPS GYTFTNYLID WVRQAPGQGLEWMG GINPGSGDTVYNEKFQ GRVTITADESTSTAYMELSSLRSEDTAVYFCAR RVRGNSFDS WGQGTLVTVSS (SEQ ID NO: 99).**

Therein the underlined parts are CDR1 (SEQ ID NO: 100), CDR2 (SEQ ID NO: 101), CDR3 (SEQ ID NO: 102) from left to right, respectively; and

The non-underlined parts are FR1 (SEQ ID NO: 103), FR2 (SEQ ID NO: 104), FR3 (SEQ ID NO: 105), and FR4 (SEQ ID NO: 106) from left to right.

Nucleic acid sequence

**CAGGTTCAGCTGGTTCAGTCTGGCGCCGAAGTGAAGAAACCTGGCAGCAG CGTGAAGGTGTCCTGCAAGCCTTCTGGCTACACCTTCACCAACTACCTGATCGA CTGGGTCCGACAGGCTCCTGGACAGGGACTTGAATGGATGGGCGGCATCAACC CTGGCAGCGGCGATACAGTGTACAACGAGAAGTTCCAGGGCAGAGTGACCATC**

**ACCGCCGACGAGTCTACCAGCACCGCCTACATGGAACTGAGCAGCCTGAGAAG CGAGGATACCGCCGTGTACTTCTGTGCCAGAAGAGTGCGGGGCAACAGCTTCG ATTCTTGGGGCCAGGGAACCCTGGTCACCGTTTCTTCT (SEQ ID NO: 196).**

18A9VHv6:

**QVQLVQSGAEVKKPGASVKVSCKPSGYAFTNYLIDWVRQAPGQGLEWMGGIN PGSGDTVYNEKFQGRVTLTADKSSSTAYMELSSLRSEDTAVYFCARRVRGNSFDSW GQGTLVTVSS (SEQ ID NO: 107).**

Therein the underlined parts are CDR1 (SEQ ID NO: 108), CDR2 (SEQ ID NO: 109), CDR3 (SEQ ID NO: 110) from left to right, respectively; and

The non-underlined parts are FR1 (SEQ ID NO: 111), FR2 (SEQ ID NO: 112), FR3 (SEQ ID NO: 113), and FR4 (SEQ ID NO: 114) from left to right, respectively.

Nucleic acid sequence

CAGGTTCAGCTGGTTCAGTCTGGCGCCGAAGTGAAGAAACCTGGCGCCAG CGTGAAGGTGTCCTGCAAGCCTTCTGGCTACGCCTTCACCAACTACCTGATCGA CTGGGTCCGACAGGCTCCTGGACAGGGACTTGAATGGATGGGCGGCATCAACC CTGGCAGCGGCGATACAGTGTACAACGAGAAGTTCCAGGGCAGAGTGACCCTG ACCGCCGACAAGTCTAGCAGCACCGCCTACATGGAACTGAGCAGCCTGAGAAG CGAGGATACCGCCGTGTACTTCTGTGCCAGAAGAGTGCGGGGCAACAGCTTCG ATTCTTGGGGCCAGGGAACCCTGGTCACCGTTTCTTCT (SEQ ID NO: 197).

18A9VHv7:

QVQLVQSGAEVKKPGASVKVSCKPS GYTFTNYLID WVRQAPGQGLEWMG GINPGSGDTVYNEKFQ GRVTLTADTSTSTVYMELSSLRSEDTAVYFCAR RVRGNSFDS WGQGTLVTVSS (SEQ ID NO: 115).

Therein, the underlined parts are CDR1 (SEQ ID NO: 116), CDR2 (SEQ ID NO: 117), CDR3 (SEQ ID NO: 118) from left to right, respectively; and

The non-underlined parts are FR1 (SEQ ID NO: 119), FR2 (SEQ ID NO: 120), FR3 (SEQ ID NO: 121), and FR4 (SEQ ID NO: 122) from left to right, respectively.

Nucleic acid sequence

CAGGTTCAGCTGGTTCAGTCTGGCGCCGAAGTGAAGAAACCTGGCGCCAG CGTGAAGGTGTCCTGCAAGCCTTCTGGCTACACCTTCACCAACTACCTGATCGA CTGGGTCCGACAGGCTCCTGGACAGGGACTTGAATGGATGGGCGGCATCAACC CTGGCAGCGGCGATACAGTGTACAACGAGAAGTTCCAGGGCAGAGTGACCCTG ACCGCCGACACCTCTACCAGCACCGTCTACATGGAACTGAGCAGCCTGAGAAG CGAGGATACCGCCGTGTACTTCTGTGCCAGAAGAGTGCGGGGCAACAGCTTCG ATTCTTGGGGCCAGGGAACCCTGGTCACCGTTTCTTCT (SEQ ID NO: 198).

Light chain variable region:
18A9VLv1:

DIVMTQSPDSLAVSLGERATINC KSSQSLLNSGNQKNYLT WYQQKPGQPPKLLIY WASTRES GVPDRFSGSGSGKDFTLTISSLQAEDVAVYYC QNNYFYPLT FGGGTKVEIK (SEQ ID NO: 123).

Therein the underlined parts are CDR1 (SEQ ID NO: 124), CDR2 (SEQ ID NO: 125), CDR3 (SEQ ID NO: 126) from left to right, respectively; and

The non-underlined parts are FR1 (SEQ ID NO: 127), FR2 (SEQ ID NO: 128), FR3 (SEQ ID NO: 129), and FR4 (SEQ ID NO: 130) from left to right, respectively.

Nucleic acid sequence

GACATCGTGATGACACAGAGCCCTGATAGCCTGGCCGTGTCTCTGGGAGA GAGAGCCACCATCAACTGCAAGAGCAGCCAGAGCCTGCTGAACAGCGGCAACC AGAAGAACTACCTGACCTGGTATCAGCAGAAGCCCGGCCAGCCTCCTAAGCTG CTGATCTACTGGGCCAGCACCAGAGAAAGCGGCGTGCCAGATAGATTCAGCGG CAGCGGCTCTGGAAAGGACTTCACCCTGACAATCAGCTCCCTGCAGGCCGAGG

ATGTGGCCGTGTACTACTGCCAGAACAACTACTTCTACCCTCTGACCTTCGGCG GAGGCACCAAGGTGGAAATCAAG (SEQ ID NO: 199).

18A9VLv2:

DIVMTQSPDSLAVSLGERATINC KSSQSLLNSGNQKNYLA WYQQKPGQPPKLLIY WASTRES GVPDRFSGSGSGTDFTLTISSLQAEDVAVYYC QQNYFYPLT FGGGTKVEIK (SEQ ID NO: 131).

Therein the underlined parts are respectively CDR1 (SEQ ID NO: 132), CDR2 (SEQ ID NO: 133), CDR3 (SEQ ID NO: 134) from left to right, respectively; and

The non-underlined parts are FR1 (SEQ ID NO: 135), FR2 (SEQ ID NO: 136), FR3 (SEQ ID NO: 137), and FR4 (SEQ ID NO: 138) from left to right, respectively.

Nucleic acid sequence

GACATCGTGATGACACAGAGCCCTGATAGCCTGGCCGTGTCTCTGGGAGA GAGAGCCACCATCAACTGCAAGAGCAGCCAGAGCCTGCTGAACAGCGGCAACC AGAAGAACTACCTGGCCTGGTATCAGCAGAAGCCCGGCCAGCCTCCTAAGCTG CTGATCTACTGGGCCAGCACCAGAGAAAGCGGCGTGCCAGATAGATTCAGCGG CAGCGGCTCTGGAACCGACTTCACCCTGACAATCAGCTCCCTGCAGGCCGAGG ATGTGGCCGTGTACTACTGCCAGCAGAACTACTTCTACCCTCTGACCTTCGGCG GAGGCACCAAGGTGGAAATCAAG (SEQ ID NO: 200).

Example 2 Pharmacological study of humanized antibodies

1. Affinity determination ($EC_{50}$) of humanized antibodies

Cells in the logarithmic growth phase were harvested, blocked with 3% BSA for 30 minutes, and plated in a U-shaped 96-well plate with $5\times10^4$ cells/100 μL. The plate was centrifuged at 1100 rpm for 3 minutes, and the supernatant was discarded. The plate was gently tapped to disperse the cells, and 50 μL of serially diluted antibodies (antibody concentrations starting from 100 nM, 8 gradients of 5-fold dilution) was added to each well and incubated at 4°C for 1 hour. After the incubation, 140 μL of 0.5% BSA was added to each well to wash three times, and 30 μL/well of AF 647/APC anti-human secondary antibody was added and incubated for 40 minutes at 4°C. After the incubation, 140 μL of 0.5% BSA was added to each well to wash three times, and finally the cells in each well were resuspended in 50 μL of PBS for FACS analysis by iQue (Intellicyt, USA) (see Table 2).

Table 2: Affinity Assay Results for Humanized Antibodies

| Antibody panel (name) | VL | VH | $EC_{50}$ (nM) |
|---|---|---|---|
| 18D10 chimera | 18D10 mVL | 18D10 mVH | 2.068 |
| h 18D10.v11 | 18010VLv1 | 18D10VHv1 | 1.131 |
| H18D 10.v12 | 18D10VLv1 | 18D10VHv2 | 1.678 |
| H18D10.v13 | 18D10VLv1 | 18D10VHv3 | 0.912 |
| H18D10.v14 | 18D10VLv1 | 18D10VHv4 | 1.744 |
| H18D10.v15 | 18D10VLv1 | 18D10VHv5 | 2.687 |
| H18D10.v16 | 18D10VLv1 | 18D10VHv6 | 2.313 |
| H18D10.v21 | 18D10VLv2 | 18D10VHv1 | 1.069 |
| H18D10.v22 | 18D10VLv2 | 18D10VHv2 | 1.897 |
| H18D10.v23 | 18D10VLv2 | 18D10VHv3 | 0.982 |

(continued)

| Antibody panel (name) | VL | VH | $EC_{50}$ (nM) |
|---|---|---|---|
| H18D10.v24 | 18D10VLv2 | 18D10VHv4 | 1.926 |
| H18D10.v25 | 18D10VLv2 | 18D10VHv5 | 1.265 |
| H18D10.v26 | 18D10VLv2 | 18D10VHv6 | 1.874 |
| 18A9 chimera | 18A9 mVL | 18A9 mVH | 1.034 |
| H18A9.v11 | 18A9VLv1 | 18A9VHv1 | 0.645 |
| H18A9.v12 | 18A9VLv1 | 18A9VHv2 | 0.913 |
| H18A9.v13 | 18A9VLv1 | 18A9VHv3 | 0.761 |
| H18A9.v14 | 18A9VLv1 | 18A9VHv4 | 0.548 |
| H18A9.v15 | 18A9VLv1 | 18A9VHv5 | 1.407 |
| H18A9.v16 | 18A9VLv1 | 18A9VHv6 | 0.799 |
| H18A9.v17 | 18A9VLv1 | 18A9VHv7 | 1.425 |
| H18A9.v21 | 18A9VLv2 | 18A9VHv1 | 1.027 |
| H18A9.v22 | 18A9VLv2 | 18A9VHv2 | 0.822 |
| H18A9.v23 | 18A9VLv2 | 18A9VHv3 | 1.170 |
| H18A9.v24 | 18A9VLv2 | 18A9VHv4 | 2.065 |
| H18A9.v25 | 18A9VLv2 | 18A9VHv5 | 0.964 |
| H18A9.v26 | 18A9VLv2 | 18A9VHv6 | 1.405 |
| H18A9.v27 | 18A9VLv2 | 18A9VHv7 | 0.646 |

2. ADCC killing activity of CM311 antibody on tumor cells

30 mL of fresh blood was taken into a 50 mL centrifuge tube, added with 15 mL of 1 × PBS, mixed well, and the mixture was slowly added to a centrifuge tube to which 20 mL of Ficoll-Paque Plus had been added, so that the blood was spread on the surface of Ficoll-Paque Plus. The centrifuge tube was centrifuged at 2000rpm for 30 minutes at 20°C, the uppermost serum was discarded, the buffy coat (i.e. PBMC) was absorbed, and separated into 50mL centrifuge tubes with 10mL per tube. No less than 30mL of 1 × PBS was added to each tube and mixed well. The tube was centrifuged at 1300 rpm for 10 minutes at 4°C, the supernatant was discarded, and the tube was added with 10 mL of 1 × PBS to rinse and count cell numbers.

The cells were resuspended in FBS/RPMI 1640 medium and cultured at 37°C in 5% $CO_2$ for 2 hours. The medium was centrifuged at 1300 rpm for 10 minutes, the supernatant was discarded, and the cells were resuspend in FBS/RPMI 1640 medium again, and inoculated in a U-shaped 96-well plate, $4\times10^5$ cells/well, 50 μL/well. The aforementioned 18D10 chimera, 18A9 chimera, anti-Claudin 18.2 humanized antibodies 18D10, 18A9 (i.e. CM311, CM311 is the collective name of humanized antibodies 18D10 or 18A9) and the dilutions of the control antibody anti-KLH (40, 20, 10, 5, 2.5, 1.25 μg/mL) were added, 25 μL/well. The plates were incubated for 30 minutes at 37°C, 5% $CO_2$. The plate was taken out and added with KATO III cells, $8\times10^3$ cells/well, 25 μL/well. The plates were incubated at 37°C with 5% $CO_2$ for 3.5 hours, added with 2 μL of 10× lysis buffer to the maximum release well of the target cells, and the incubation was continued at 37°C with 5% $CO_2$ for 30 minutes. The plate was taken out, centrifuged at 1000rpm for 3 minutes, and the supernatant was transferred to a black ELISA plate, 50 μL/well. 50μL/well of LDH detection substrate was added, incubated at room temperature for 10 minutes, and added with 25 μL/well of stop solution to stop the reaction. The OD (optical density) was tested by a microplate reader (Biotek).

The result is calculated as follows:

$$\%\ \text{cell killing} = (OD_{\text{experimental}} - OD_{\text{control release}}) / (OD_{\text{maximum release}} - OD_{\text{control release}}) \times 100\%$$

As shown in Fig. 4, the anti-Claudin 18.2 humanized antibodies, 18D10 and 18A9 have strong ADCC activity.

3. CDC cell killing activity of CM311 antibody on tumor cells

KATO III cells in logarithmic growth phase were resuspended in $1\times10^7$ cells/mL. CFSE (Sigma, 87444-5MG-F) was added at a final concentration of 1 μM. Incubate at room temperature for 10 minutes, and add 3 volumes of medium to stop the reaction. Centrifuge at 1000 rpm at 4°C for 5 minutes, discard the supernatant, resuspend in the medium, and seed the cells into a 96-well plate, $1\times10^5$ cells/well, 50 μL/well. Add the diluted aforementioned 18D10 chimera, 18A9 chimera, anti-Claudin 18.2 humanized antibodies 18D10, 18A9 (CM311) and control antibody anti-KLH (the diluted concentrations are 30, 10, 3.33, 1.11, 0.37 μg/mL), 50 μL/well. The complement was diluted to 30% with medium, and added to the plate, 50 μL/well. Incubate at 37°C, 5% $CO_2$ for 2 hours, centrifuge at 1000 rpm for 3 minutes, and discard the supernatant. PI staining solution (1:200 dilution) was mixed with Sulfate latex (Invitrogen, S37227), and then added to 96-well plate, 100 μL/well. Incubate on ice for 10 minutes, and then analyze the cells by FACS. As shown in Fig. 5, the anti-Claudin 18.2 humanized antibodies 18D10 and 18A9 have strong CDC effects.

Example 3 Preparation and Analysis of antibody-drug conjugates

1. Preparation of antibody-drug conjugate

10 mg of CM311 antibody was buffer exchanged using a 15 mL 30 KD ultrafiltration device into a reduction buffer (25 mM sodium borate, pH 8.0, 25 mM NaCl, 5 mM EDTA) for a total of three times; the final volume was about 1 mL, transferred to a new Eppendorf centrifuge tube (weighed), and weighed; the protein concentration was measured and the total amount of protein was calculated. 2.5 times molar amount of DTT was added to the antibody and incubated at room temperature for 2 hours and continuously mixed. The mixture was buffer exchanged using a 15 ml 30 KD ultrafiltration device into a coupling buffer (50 mM Tris, pH 7.2, 150 mM NaCl, 5 mM EDTA) for a total of three times. The solution was taken, measured by A280 to determine protein concentration, and weighed, and the total amount of protein was calculated. 10 μL of sample was taken and measured by Ellman's method to determine number of free thiol groups.

In addition, the molar concentration of its free thiol groups was calculated by the following formula:

$$C_{thiol} = \frac{A412 \times 112}{b \times 14150}(M)$$

b: optical path length of cuvette (usually 1 cm).

The mole number of free thiol groups was calculated from the molar concentration of free thiol groups and the volume of total protein solution.

To the reduced antibody was added 1.1 times the mole number of free thiol groups of vc-MMAE (i.e. MC-vc-PAB-MMAE) (dissolved in DMSO), mixed at room temperature and reacted for 2 hours, intermittently mixed. To the reaction system was added with N-acetylcysteine in an amount of 20 times the mole number of vc-MMAE (i.e. MC-vc-PAB-MMAE) in the reaction solution, mixed, and the mixture was allowed to stand for 5 minutes. The mixture was buffer exchanged using a 15 ml 30 KD ultrafiltration device into a conjugate stock solution (20mM Histidine, 3% Sucrose, 0.03% Tween-80, pH 5.5) for a total of 3 times. The obtained antibody-drug conjugate product Anti-Claudin-18.2-ADC (a term used collectively for antibody-drug conjugates prepared from humanized antibody 18D10 or 18A9) was stored at 4°C.

2. DAR determination of antibody-drug conjugates

The drug loading (drug-to-antibody ratio, DAR) of the antibody-drug conjugates was determined by hydrophobic interaction chromatography (HIC-HPLC).

The HIC profile of a typical antibody-drug conjugate CM311-18D10-VH6/VL1-ADC was shown in Fig. 6. According to the peak area of the profile, the average DAR was 3.8.

Similarly, the average DAR of the antibody-drug conjugate CM311-18D10-VH3/VL2-ADC was 3.5.

The average DAR of the antibody-drug conjugate CM311-18D10-VH6/VL2-ADC was 3.4.

The average DAR of the antibody-drug conjugate CM311-18A9-VH7/VL2-ADC was 3.0.

It should be noted that the names of the above antibody-drug conjugates, like CM311-18D10-VH6/VL1-ADC, indicate that the antibody used in the preparation of the antibody-drug conjugate is CM311-18D10-VH6/VL1. Further, the antibody name CM311-18D10-VH6/VL1 indicates that the antibody used in the preparation of the aforementioned antibody-drug conjugates is the one in a class of 18D10 antibodies with the variable region of the heavy chain being VHv6 and the variable region of the light chain being VLv1; As can be seen from Table 2 above, the specific antibody used in the preparation of the aforementioned antibody-drug conjugate is h18D10.v16. The names of the remaining

three antibody-drug conjugates CM311-18D10-VH3/VL2-ADC, CM311-18D10-VH6/VL2-ADC and CM311-18A9-VH7/VL2-ADC can be understood by referring to the aforementioned. Specifically, the specific antibody used in the preparation of the antibody-drug conjugate CM311-18D10-VH3/VL2-ADC was h18010.v23 in Table 2; the specific antibody used in the preparation of the antibody-drug conjugate CM311-18D10-VH6/VL2-ADC is h18D10.v26 in Table 2; the specific antibody used in the preparation of the antibody-drug conjugate CM311-18A9-VH7/VL2-ADC is h18A9.v27 in Table 2.

Example 4 *In vitro* pharmacodynamic study of antibody-drug conjugates

After 1-2 passages of the recovered cell lines, firstly the supernatant was pipetted into a 15 mL centrifuge tube, centrifuged, and the obtained supernatant was discarded; the cell culture flask was rinsed with 5 mL of PBS, and then the cells were digested with 2 mL of Trypsin-EDTA. The cells were resuspended with medium in the previous 15 mL centrifuge tube, and centrifuged; the supernatant was discarded, the cells were resuspended with medium again, and 0.5 mL was taken out and counted with a cell counter. Cells were plated on 96-well cell culture plates (LT-1C8 cells at 5000 or 10000 cells/well, LT-M11 cells at 5000 cells/well, and BxPC-3 at 3000 cells/well); after 24 hours of culture, serially diluted concentrations of antibody-drug conjugate CM311 ADCs (conjugated by different CM311 monoclonal antibodies) were added and incubated for 96 hours, then CCK-8 or Presto-Blue detection reagent was added to each well, and a microplate reader was used for detection and four parameter fitting was carried out.
Experimental reagents and sources:

Articles

| Antibody-Drug Conjugate (Name) | Concentration | Storage conditions |
|---|---|---|
| CM311-18D10-VH3/VL2-ADC | 1.0 mg/mL | 2-8°C |
| CM311-18D10-VH6/VL1-ADC | 1.2 mg/mL | 2-8°C |
| CM311-18D10-VH6/VL2-ADC | 0.9 mg/mL | 2-8°C |
| CM311-18A9-VH7/VL2-ADC | 0.7 mg/mL | 2-8°C |

Cell lines

| Cell line | Cell type | Claudin 18.2 expression level | Origin | Culture medium |
|---|---|---|---|---|
| LT-M11 | KATO stable cell line | High expression | KEYMED BIOSCIENCES CO LTD | IMDM + 20% FBS |
| LT-1C8 | KATO stable cell line | Medium expression | KEYMED BIOSCIENCES CO LTD. | IMDM + 20% FBS |

Experimental results:

The average value of $IC_{50}$ of each CM311 ADC was shown in Table 3. Fig. 7 is a representative graph of the cell killing effect on LT-M11 cells by different CM311 ADCs.

Table 3: $IC_{50}$ Values of Different CM311 ADCs in Screened Cell Lines

| Cell line | CM311-18D10-VH3/VL2-ADC Mean $IC_{50}$ (ng/mL) | CM311-18D10-VH6/VL1-ADC Mean $IC_{50}$ (ng/mL) | CM311-18D10-VH6/VL2-ADC Mean $IC_{50}$ (ng/mL) | CM311-18A9-VH7/VL2-ADC Mean $IC_{50}$ (ng/mL) |
|---|---|---|---|---|
| LT-1C8 | 520.5 | 253.9 | 615.1 | 111.1 |

(continued)

| Cell line | CM311-18D10-VH3/VL2-ADC Mean $IC_{50}$ (ng/mL) | CM311-18D10-VH6/VL1-ADC Mean $IC_{50}$ (ng/mL) | CM311-18D10-VH6/VL2-ADC Mean $IC_{50}$ (ng/mL) | CM311-18A9-VH7/VL2-ADC Mean $IC_{50}$ (ng/mL) |
|---|---|---|---|---|
| LT-M11 | 200.3 | 113.8 | 349.6 | 155.5 |
| | 131.5 | 51.07 | 146.6 | 51.22 |
| BxPC-3 | 5295 | 4632 | 4237 | 4045 |
| Note: In Fig. 7: | | | | |

Sample 1: CM311-18D10-VH3/VL2-ADC
Sample 2: CM311-18D10-VH6/VL1-ADC
Sample 3: CM311-18D10-VH6/VL2-ADC
Sample 4: CM311-18A9-VH7/VL2-ADC

It can be seen from the results in Table 3 and Fig. 7 that different CM311 ADCs showed significant cell killing activity in cell lines moderately and highly expressing Claudin 18.2, but no significant cell killing activity in Claudin 18.2-negative BxPC-3 cell line.

Example 5 In vivo pharmacodynamic study of antibody-drug conjugates

The antitumor activity of CM311ADC was tested in two gastric cancer PDX models, STO#025 and STO#523. Both gastric cancer models had higher Claudin 18 mRNA levels.

The process of establishing the PDX models of human gastric cancer in nude mice was as follows: tumor tissue with a volume of about 15-30 $mm^3$ was transplanted into the back of BALB/c nude mice subcutaneously. When the tumor volume reached 150-260 $mm^3$, the mice were randomly divided into groups, with 5 mice in each group, to ensure that the tumor volume was uniform among different groups and the body weight was taken into consideration as well. There were 4 groups in total, including the vehicle group, 1 mg/kg CM311-ADC-1 administration group, 3 mg/kg CM311-ADC-1 administration group and 3 mg/kg CM311-ADC-2 (non-binding, control ADC) administration group. Among them, CM311-ADC-1 refers to the antibody drug conjugate CM311-18D10-VH6/VL1-ADC; compared with CM311-ADC-1, the antibody of CM311-ADC-2 is a human IgG1 isotype control, which does not bind to the target on the surface of tumor cells.

Data Analysis: During the experiment, tumor volumes were measured twice a week. The formula for calculating tumor volume (TV) is: $TV = l \times w^2/2$, wherein l and w represent the measured length and width of the tumor, respectively. The relative tumor volume (RTV) was calculated according to the measurement results, $RTV = V_f/V0$, wherein V0 is the tumor volume measured at the time of group administration (i.e., Day 0), and $V_f$ is the tumor volume measured on the last day. Relative tumor proliferation rate T/C (%) = (RTV in administration group/RTV in Vehicle group)$\times$100%. Tumor growth inhibition rate TGI% = (average tumor volume of vehicle group-average tumor volume of administration group)/average tumor volume of vehicle group$\times$100%. When T/C(%) $\leq$ 40%, and $P < 0.05$, it is considered that the test ADC has a significant inhibitory effect on tumor growth.

Experimental results:

1) Efficacy study of CM311ADC in human gastric cancer PDX model STO#025 with high Claudin 18 mRNA expression

The experimental results were shown in Fig. 8 and Fig. 9. After administration of CM311-ADC-1 at 1 and 3 mg/kg, the relative tumor proliferation rate T/C (%) on Day 28 was 29.86% and 0%, respectively, and the tumor growth inhibition rate TGI% was 70.13% and 100%, respectively, with 0/5 and 5/5 tumors completely regressed, and 2/5 and 0/5 tumors partially regressed, respectively; T/C (%) of CM311-ADC-2 (3 mg/kg) group was 67.24%, and TGI % was 32.76%. The experimental results showed that both CM311-ADC-1 (3 mg/kg) and CM311-ADC-1 (1 mg/kg) had significant inhibitory activity on tumor growth, while CM311-ADC-2 (3 mg/kg) had no significant antitumor activity. The tumor-bearing mice can tolerate CM311-ADC-1 and CM311-ADC-2 very well.

2) Efficacy study of CM311ADC in human gastric cancer PDX model STO#523 with high Claudin 18 mRNA expression

The experimental results were shown in Fig. 10 and Fig. 11. After administration of CM311-ADC-1 at 1 and 3

mg/kg, the relative tumor proliferation rate T/C (%) on Day 28 was 35.60% and 6.79%, respectively, and the tumor growth inhibition rate TGI% was 64.40% and 93.21%, respectively; T/C (%) of CM311-ADC-2 (3 mg/kg) group was 114.81%, and TGI% was -14.81%. The experimental results showed that both CM311-ADC-1 (3 mg/kg) and CM311-ADC-1 (1 mg/kg) had significant inhibitory activity against tumor growth, while CM311-ADC-2 (3 mg/kg) had no significant anti-tumor activity. The tumor-bearing mice can tolerate CM311-ADC-1 and CM311-ADC-2 very well.

Example 6 *In vitro* cellular activity comparison of antibody and antibody-drug conjugates

Testing method:

The KATO III cell line was plated at 5000 cells/well, and the samples to be tested were added 24 hours later. All samples to be tested were diluted 2.4 folds starting from a final concentration of 1000 ng/mL for 9 times and incubated for 96 hours, and the fluorescence intensity was read by a microplate reader after color development for 60 min with PrestoBlue.

The samples to be tested were as follows:

Sample1: CM311, specifically CM311-18D10-VH6/VL1;
Sample2: Anti-Claudin-18.2-ADC, specifically CM311-18D10-VH6/VL1-ADC;
Sample 3: Non-binding, control ADC, specifically IgG-L1D1 (IgG-L1D1 is an ADC in which an IgG antibody is conjugated with a L1D1 small molecule (i.e. vcMMAE, that is, MC-vc-PAB-MMAE).

The test results were shown in Fig. 12 and Table 4. As can be seen from Fig. 12, Anti-Claudin-18.2-ADC (e.g. CM311-18D10-VH6/VL1-ADC) showed strong cell killing with an $EC_{50}$ value of 16.37 ng/mL; while CM311 (e.g. CM311-18D10 -VH6/VL1) and IgG-L1D1 did not show significant cell killing effect.

**Table 4: Comparative results of *in vitro* cell activity of antibodies and antibody-drug conjugates (4-parameter fitting results)**

Curve Fit : 4-Parameter $y = D + \frac{A - D}{1 + \left(\frac{x}{C}\right)^B}$

| | Parameter | Estimated Value | Std. Error | Confidence Interval |
|---|---|---|---|---|
| CM311 $R^2$ = 0.939 EC50 = 1.29e+13 | A | 4.087 | 0.017 | [4.046, 4.127] |
| | B | 0.516 | 0.571 | [-0.881, 1.913] |
| | C | 1.29e+13 | 6.41e+18 | [-1.57e+19, 1.57e+19] |
| | D | -2.42e+4 | 6.19e+9 | [-1.51e+10, 1.51e+10] |
| Anti-Claudin-18.2-ADC $R^2$ = 0.997 EC50 = 16.37 | A | 4.107 | 0.017 | [4.066, 4.148] |
| | B | 1.437 | 0.149 | [1.073, 1.801] |
| | C | 16.37 | 1.287 | [13.22, 19.52] |
| | D | 3.337 | 0.015 | [3.299, 3.375] |
| IgG-L1D1 $R^2$ = 0.980 EC50 = 9.04e+12 | A | 4.098 | 0.013 | [4.065, 4.130] |
| | B | 0.555 | 0.330 | [-0.253, 1.362] |
| | C | 9.04e+12 | 4.62e+18 | [-1.13e+19, 1.13e+19] |
| | D | -7.86e+4 | 2.23e+10 | [-5.45e+10, 5.45e+10] |

## Claims

1. Antibody drug conjugate, its pharmaceutically acceptable salt, solvate or solvate of said salt, the antibody drug conjugate has the structure shown in Formula I,

$$Ab\text{-}(L\text{-}D)_p \qquad \text{Formula I}$$

wherein:

Ab is an anti-Claudin 18.2 antibody, the anti-Claudin 18.2 antibody comprises a heavy chain and a light chain, and the heavy chain variable region CDR1 comprises a sequence selected from a sequence as shown in SEQ ID NO: 2, 10, 18, 26, 34, 42, 68, 76, 84, 92, 100, 108 or 116 or a mutant thereof, the heavy chain variable region CDR2 comprises a sequence selected from a sequence as shown in SEQ ID NO: 3, 11, 19, 27, 35, 43, 69, 77, 85, 93, 101, 109 or 117 or a mutant thereof, the heavy chain variable region CDR3 comprises a sequence selected from a sequence as shown in SEQ ID NO: 4, 12, 20, 28, 36, 44, 70, 78, 86, 94, 102, 110 or 118 or a mutant thereof, the light chain variable region CDR1 comprises a sequence selected from a sequence as shown in SEQ ID NO: 50, 58, 124 or 132 or a mutant thereof, the light chain variable region CDR2 comprises a sequence selected from a sequence as shown in SEQ ID NO: 51, 59, 125 or 133 or a mutant thereof, and the light chain variable region CDR3 comprises a sequence selected from a sequence as shown in SEQ ID NO: 52, 60, 126 or 134 or a mutant thereof;
D is cytotoxic agent;
L is the linker for linking the anti-Claudin 18.2 antibody and the cytotoxic agent;
p is 2.0-8.0.

**2.** The antibody drug conjugate of claim 1, its pharmaceutically acceptable salt, solvate or solvate of said salt, wherein:

the heavy chain variable region CDR1 of the anti-Claudin 18.2 antibody comprises a sequence selected from a sequence as shown in SEQ ID NO: 2, 10, 18, 26, 34 or 42 or a mutant thereof, the heavy chain variable region CDR2 comprises a sequence selected from a sequence as shown in SEQ ID NO: 3, 11, 19, 27, 35 or 43 or a mutant thereof, the heavy chain variable region CDR3 comprises a sequence selected from a sequence as shown in SEQ ID NO: 4, 12, 20, 28, 36 or 44 or a mutant thereof, the light chain variable region CDR1 comprises a sequence selected from a sequence as shown in SEQ ID NO: 50 or 58 or a mutant thereof, the light chain variable region CDR2 comprises a sequence selected from a sequence as shown in SEQ ID NO: 51 or 59 or a mutant thereof, and the light chain variable region CDR3 comprises a sequence selected from a sequence as shown in SEQ ID NO: 52 or 60 or a mutant thereof; or
the heavy chain variable region CDR1 of the anti-Claudin 18.2 antibody comprises a sequence selected from a sequence as shown in SEQ ID NO: 68, 76, 84, 92, 100, 108 or 116 or a mutant thereof, the heavy chain variable region CDR2 comprises a sequence selected from a sequence as shown in SEQ ID NO: 69, 77, 85, 93, 101, 109 or 117 or a mutant thereof, the heavy chain variable region CDR3 comprises a sequence selected from a sequence as shown in SEQ ID NO: 70, 78, 86, 94 102, 110 or 118 or a mutant thereof, the light chain variable region CDR1 comprises a sequence selected from a sequence as shown in SEQ ID NO: 124 or 132 or a mutant thereof, the light chain variable region CDR2 comprises a sequence selected from a sequence as shown in SEQ ID NO: 125 or 133 or a mutant thereof, and the light chain variable region CDR3 comprises a sequence selected from a sequence as shown in SEQ ID NO: 126 or 134 or a mutant thereof; or
the heavy chain variable region CDR1, CDR2 and CDR3 of the anti-Claudin 18.2 antibody are selected from the following sequence combinations:

(1) SEQ ID NO: 2, SEQ ID NO: 3, SEQ ID NO: 4,
(2) SEQ ID NO: 10, SEQ ID NO: 11, SEQ ID NO: 12,
(3) SEQ ID NO: 18, SEQ ID NO: 19, SEQ ID NO: 20,
(4) SEQ ID NO: 26, SEQ ID NO: 27, SEQ ID NO: 28,
(5) SEQ ID NO: 34, SEQ ID NO: 35, SEQ ID NO: 36,
(6) SEQ ID NO: 42, SEQ ID NO: 43, SEQ ID NO: 44,
(7) SEQ ID NO: 68, SEQ ID NO: 69, SEQ ID NO: 70,
(8) SEQ ID NO: 76, SEQ ID NO: 77, SEQ ID NO: 78,
(9) SEQ ID NO: 84, SEQ ID NO: 85, SEQ ID NO: 86,
(10) SEQ ID NO: 92, SEQ ID NO: 93, SEQ ID NO: 94,
(11) SEQ ID NO: 100, SEQ ID NO: 101, SEQ ID NO: 102,
(12) SEQ ID NO: 108, SEQ ID NO: 109, SEQ ID NO: 110,
(13) SEQ ID NO: 116, SEQ ID NO: 117, SEQ ID NO: 118,

the light chain variable region CDR1, CDR2 and CDR3 of the anti-Claudin 18.2 antibody are selected from the following sequence combinations:

(1) SEQ ID NO: 50, SEQ ID NO: 51, SEQ ID NO: 52,
(2) SEQ ID NO: 58, SEQ ID NO: 59, SEQ ID NO: 60,
(3) SEQ ID NO: 124, SEQ ID NO: 125, SEQ ID NO: 126,

(4) SEQ ID NO: 132, SEQ ID NO: 133, SEQ ID NO: 134;

preferably, the heavy chain variable region CDR1, CDR2 and CDR3 of the anti-Claudin 18.2 antibody are selected from the following sequence combinations:

(1) SEQ ID NO: 2, SEQ ID NO: 3, SEQ ID NO: 4,
(2) SEQ ID NO: 10, SEQ ID NO: 11, SEQ ID NO: 12,
(3) SEQ ID NO: 18, SEQ ID NO: 19, SEQ ID NO: 20,
(4) SEQ ID NO: 26, SEQ ID NO: 27, SEQ ID NO: 28,
(5) SEQ ID NO: 34, SEQ ID NO: 35, SEQ ID NO: 36,
(6) SEQ ID NO: 42, SEQ ID NO: 43, SEQ ID NO: 44,

the light chain variable region CDR1, CDR2 and CDR3 of the anti-Claudin 18.2 antibody are selected from the following sequence combinations:

(1) SEQ ID NO: 50, SEQ ID NO: 51, SEQ ID NO: 52,
(2) SEQ ID NO: 58, SEQ ID NO: 59, SEQ ID NO: 60; or

preferably, the heavy chain variable region CDR1, CDR2 and CDR3 of the anti-Claudin 18.2 antibody are selected from the following sequence combinations:

(1) SEQ ID NO: 68, SEQ ID NO: 69, SEQ ID NO: 70,
(2) SEQ ID NO: 76, SEQ ID NO: 77, SEQ ID NO: 78,
(3) SEQ ID NO: 84, SEQ ID NO: 85, SEQ ID NO: 86,
(4) SEQ ID NO: 92, SEQ ID NO: 93, SEQ ID NO: 94,
(5) SEQ ID NO: 100, SEQ ID NO: 101, SEQ ID NO: 102,
(6) SEQ ID NO: 108, SEQ ID NO: 109, SEQ ID NO: 110,
(7) SEQ ID NO: 116, SEQ ID NO: 117, SEQ ID NO: 118,

the light chain variable region CDR1, CDR2 and CDR3 of the anti-Claudin 18.2 antibody are selected from the following sequence combinations:

(1) SEQ ID NO: 124, SEQ ID NO: 125, SEQ ID NO: 126,
(2) SEQ ID NO: 132, SEQ ID NO: 133, SEQ ID NO: 134.

**3.** The antibody drug conjugate of claim 1, its pharmaceutically acceptable salt, solvate or solvate of said salt, wherein:

the heavy chain variable region FR1 of the anti-Claudin 18.2 antibody comprises a sequence selected from a sequence as shown in SEQ ID NO: 5, 13, 21, 29, 37, 45, 71, 79, 87, 95, 103, 111 or 119 or a mutant thereof, the heavy chain variable region FR2 comprises a sequence selected from a sequence as shown in SEQ ID NO: 6, 14, 22, 30, 38, 46, 72, 80, 88, 96, 104, 112 or 120 or a mutant thereof, the heavy chain variable region FR3 comprises a sequence selected from a sequence as shown in SEQ ID NO: 7, 15, 23, 31, 39, 47, 73, 81, 89, 97, 105, 113 or 121 or a mutant thereof, the heavy chain variable region FR4 comprises a sequence selected from a sequence as shown in SEQ ID NO: 8, 16, 24, 32, 40, 48, 74, 82, 90, 98, 106, 114 or 122 or a mutant thereof, the light chain variable region FR1 comprises a sequence selected from a sequence as shown in SEQ ID NO: 53, 61, 127 or 135 or a mutant thereof, the light chain variable region FR2 comprises a sequence selected from a sequence as shown in SEQ ID NO: 54, 62, 128 or 136 or a mutant thereof, the light chain variable region FR3 comprises a sequence selected from a sequence as shown in SEQ ID NO: 55, 63, 129 or 137 or a mutant thereof, and the light chain variable region FR4 comprises a sequence selected from a sequence as shown in SEQ ID NO: 56, 64, 130 or 138 or a mutant thereof;
preferably, the heavy chain variable region FR1 of the anti-Claudin 18.2 antibody comprises a sequence selected from a sequence as shown in SEQ ID NO: 5, 13, 21, 29, 37 or 45 or a mutant thereof, the heavy chain variable region FR2 comprises a sequence selected from a sequence as shown in SEQ ID NO: 6,14, 22, 30, 38 or 46 or a mutant thereof, the heavy chain variable region FR3 comprises a sequence selected from a sequence as shown in SEQ ID NO: 7, 15, 23, 31, 39 or 47 or a mutant thereof, the heavy chain variable region FR4 comprises a sequence selected from a sequence as shown in SEQ ID NO: 8, 16, 24, 32, 40 or 48 or a mutant thereof, the light chain variable region FR1 comprises a sequence a selected from a sequence as shown in SEQ ID NO: 53 or 61, or a mutant thereof, the light chain variable region FR2 comprises a sequence selected from a sequence

as shown in SEQ ID NO: 54 or 62 or a mutant thereof, the light chain variable region FR3 comprises a sequence selected from a sequence as shown in SEQ ID NO: 55 or 63 or a mutant thereof, and the light chain variable region FR4 comprises a sequence selected from a sequence as shown in SEQ ID NO: 56 or 64 or a mutant thereof; or

preferably, the heavy chain variable region FR1 of the anti-Claudin 18.2 antibody comprises a sequence selected from a sequence as shown in SEQ ID NO: 71, 79, 87, 95, 103, 111 or 119 or a mutant thereof, the heavy chain variable region FR2 comprises a sequence selected from a sequence as shown in SEQ ID NO: 72, 80, 88, 96, 104, 112 or 120 or a mutant thereof, the heavy chain variable region FR3 comprises a sequence selected from a sequence as shown in SEQ ID NO: 73, 81, 89, 97, 105, 113 or 121 or a mutant thereof, the heavy chain variable region FR4 comprises a sequence selected from a sequence as shown in SEQ ID NO: 74, 82, 90, 98, 106, 114 or 122 or a mutant thereof, the light chain variable region FR1 comprises a sequence selected from a sequence as shown in SEQ ID NO: 127 or 135 or a mutant thereof, the light chain variable region FR2 comprises a sequence selected from SEQ ID NO: 128 or 136 or a mutant thereof, the light chain variable region FR3 comprises a sequence selected from a sequence as shown in SEQ ID NO: 129 or 137 or a mutant thereof, and the light chain variable region FR4 comprises a sequence selected from a sequence as shown in SEQ ID NO: 130 or 138 or a mutant thereof; or

preferably, the heavy chain variable region FR1, FR2, FR3, FR4 of the anti-Claudin 18.2 antibody are selected from the following sequence combinations:

(1) SEQ ID NO: 5, SEQ ID NO: 6, SEQ ID NO: 7, SEQ ID NO: 8,
(2) SEQ ID NO: 13, SEQ ID NO: 14, SEQ ID NO: 15, SEQ ID NO: 16,
(3) SEQ ID NO: 21, SEQ ID NO: 22, SEQ ID NO: 23, SEQ ID NO: 24,
(4) SEQ ID NO: 29, SEQ ID NO: 30, SEQ ID NO: 31, SEQ ID NO: 32,
(5) SEQ ID NO: 37, SEQ ID NO: 38, SEQ ID NO: 39, SEQ ID NO: 40,
(6) SEQ ID NO:45, SEQ ID NO:46, SEQ ID NO:47, SEQ ID NO:48,
(7) SEQ ID NO:71, SEQ ID NO:72, SEQ ID NO:73, SEQ ID NO:74,
(8) SEQ ID NO: 79, SEQ ID NO: 80, SEQ ID NO: 81, SEQ ID NO: 82,
(9) SEQ ID NO: 87, SEQ ID NO: 88, SEQ ID NO: 89, SEQ ID NO: 90,
(10) SEQ ID NO: 95, SEQ ID NO: 96, SEQ ID NO: 97, SEQ ID NO: 98,
(11) SEQ ID NO: 103, SEQ ID NO: 104, SEQ ID NO: 105, SEQ ID NO: 106,
(12) SEQ ID NO: 111, SEQ ID NO: 112, SEQ ID NO: 113, SEQ ID NO: 114,
(13) SEQ ID NO: 119, SEQ ID NO: 120, SEQ ID NO: 121, SEQ ID NO: 122,

the light chain variable region FR1, FR2, FR3 and FR4 of the anti-Claudin 18.2 antibody are selected from the following sequence combinations:

(1) SEQ ID NO: 53, SEQ ID NO: 54, SEQ ID NO: 55, SEQ ID NO: 56,
(2) SEQ ID NO: 61, SEQ ID NO: 62, SEQ ID NO: 63, SEQ ID NO: 64,
(3) SEQ ID NO: 127, SEQ ID NO: 128, SEQ ID NO: 129, SEQ ID NO: 130,
(4) SEQ ID NO: 135, SEQ ID NO: 136, SEQ ID NO: 137, SEQ ID NO: 138;

more preferably, the heavy chain variable region FR1, FR2, FR3 and FR4 of the anti-Claudin 18.2 antibody are selected from the following sequence combinations:

(1) SEQ ID NO: 5, SEQ ID NO: 6, SEQ ID NO: 7, SEQ ID NO: 8,
(2) SEQ ID NO: 13, SEQ ID NO: 14, SEQ ID NO: 15, SEQ ID NO: 16,
(3) SEQ ID NO: 21, SEQ ID NO: 22, SEQ ID NO: 23, SEQ ID NO: 24,
(4) SEQ ID NO: 29, SEQ ID NO: 30, SEQ ID NO: 31, SEQ ID NO: 32,
(5) SEQ ID NO: 37, SEQ ID NO: 38, SEQ ID NO: 39, SEQ ID NO: 40,
(6) SEQ ID NO:45, SEQ ID NO:46, SEQ ID NO:47, SEQ ID NO:48,

the light chain variable region FR1, FR2, FR3 and FR4 of the anti-Claudin 18.2 antibody are selected from the following sequence combinations:

(1) SEQ ID NO: 53, SEQ ID NO: 54, SEQ ID NO: 55, SEQ ID NO: 56,
(2) SEQ ID NO: 61, SEQ ID NO: 62, SEQ ID NO: 63, SEQ ID NO: 64; or

more preferably, the heavy chain variable region FR1, FR2, FR3 and FR4 of the anti-Claudin 18.2 antibody are

selected from the following sequence combinations:

(1) SEQ ID NO:71, SEQ ID NO:72, SEQ ID NO:73, SEQ ID NO:74,
(2) SEQ ID NO: 79, SEQ ID NO: 80, SEQ ID NO: 81, SEQ ID NO: 82,
(3) SEQ ID NO: 87, SEQ ID NO: 88, SEQ ID NO: 89, SEQ ID NO: 90,
(4) SEQ ID NO: 95, SEQ ID NO: 96, SEQ ID NO: 97, SEQ ID NO: 98,
(5) SEQ ID NO: 103, SEQ ID NO: 104, SEQ ID NO: 105, SEQ ID NO: 106,
(6) SEQ ID NO: 111, SEQ ID NO: 112, SEQ ID NO: 113, SEQ ID NO: 114,
(7) SEQ ID NO: 119, SEQ ID NO: 120, SEQ ID NO: 121, SEQ ID NO: 122,

the light chain variable region FR1, FR2, FR3 and FR4 of the anti-Claudin 18.2 antibody are selected from the following sequence combinations:

(1) SEQ ID NO: 127, SEQ ID NO: 128, SEQ ID NO: 129, SEQ ID NO: 130,
(2) SEQ ID NO: 135, SEQ ID NO: 136, SEQ ID NO: 137, SEQ ID NO: 138.

**4.** The antibody drug conjugate of claim 1, its pharmaceutically acceptable salt, solvate or solvate of said salt, wherein:

the heavy chain variable region of the anti-Claudin 18.2 antibody is selected from a sequence as shown in SEQ ID NO: 1, 9, 17, 25, 33, 41, 67, 75, 83, 91, 99, 107 or 115,
the light chain variable region of the anti-Claudin 18.2 antibody is selected from a sequence as shown in SEQ ID NO: 49, 57, 123 or 131;
preferably, the heavy chain variable region of the anti-Claudin 18.2 antibody is selected from a sequence as shown in SEQ ID NO: 1, 9, 17, 25, 33 or 41,
the light chain variable region of the anti-Claudin 18.2 antibody is selected from a sequence as shown in SEQ ID NO: 49 or 57; or
preferably, the heavy chain variable region of the anti-Claudin 18.2 antibody is selected from a sequence as shown in SEQ ID NO: 67, 75, 83, 91, 99, 107 or 115;
the light chain variable region of the anti-Claudin 18.2 antibody is selected from a sequence as shown in SEQ ID NO: 123 or 131.

**5.** The antibody drug conjugate of claim 1, its pharmaceutically acceptable salt, solvate or solvate of said salt, wherein:

the heavy chain variable region and the light chain variable region of the anti-Claudin 18.2 antibody are selected from the following sequence combinations:

(1) SEQ ID NO: 17 and SEQ ID NO: 57,
(2) SEQ ID NO: 41 and SEQ ID NO: 49,
(3) SEQ ID NO: 41 and SEQ ID NO: 57,
(4) SEQ ID NO: 115 and SEQ ID NO: 131;

preferably, the sequences of the heavy chain variable region and the light chain variable region of the anti-Claudin 18.2 antibody are SEQ ID NO: 41 and SEQ ID NO: 49, respectively.

**6.** The antibody drug conjugate of claim 1, its pharmaceutically acceptable salt, solvate- or solvate of said salt, wherein:

the heavy chain constant region of the anti-Claudin 18.2 antibody is selected from human IgG (such as IgG1, IgG2, IgG3 or IgG4), IgM, IgA, IgD, IgA constant region or a mutant of the above constant region, preferably human IgG1;
the light chain constant region of the anti-Claudin 18.2 antibody is selected from a human lambda constant region, a kappa constant region or a mutant of the above constant region, preferably a human kappa constant region.

**7.** The antibody drug conjugate of claim 1, its pharmaceutically acceptable salt, solvate or solvate of said salt, wherein:

the amino acid sequence of the heavy chain of the anti-Claudin 18.2 antibody comprises a sequence as shown in SEQ ID NO: 65, or a sequence having greater than 70%, such as greater than 75%, 80%, 85%, 90%, 95%, 99% identity to SEQ ID NO: 65;

the amino acid sequence of the light chain of the anti-Claudin 18.2 antibody comprises a sequence as shown in SEQ ID NO: 66, or a sequence having greater than 70%, such as greater than 75%, 80%, 85%, 90%, 95%, 99% identity to SEQ ID NO: 66.

**8.** The antibody drug conjugate of claim 1, its pharmaceutically acceptable salt, solvate or solvate of said salt, wherein p is 2.0-7.0, 2.0-6.0, 2.0-5.0, 2.0-4.0, 3.0-7.0, 3.0-6.0, 3.0-5.0 or 3.0-4.0, preferably p is 3.0-4.0, e.g. p is 3.0-3.8, preferably 3.0, 3.4, 3.5 or 3.8, more preferably 3.8.

**9.** The antibody drug conjugate of claim 1, its pharmaceutically acceptable salt, solvate or solvate of said salt, wherein the cytotoxic agent is selected from SN-38, Gemcitabine, Monomethyl auristatin E (MMAE), Monomethyl auristatin F (MMAF), maytansinoids (e.g. Maytansine DM1, Maytansine DM4), calicheamicin, MGBA (e.g. duocarmycin), doxorubicin, ricin, Diphtheria toxin and other toxins, I131, interleukins, tumor necrosis factor, chemokines and nanoparticles;
preferably, the cytotoxic agent is MMAE.

**10.** The antibody drug conjugate of claim 1, its pharmaceutically acceptable salt, solvate or solvate of said salt, wherein the linker is selected from 6-maleimidocaproyl (MC), maleimidopropionyl (MP), N-succinimidyl 4-(2-pyridylthio)valerate (SPP), 4-(N-maleimidomethyl)-cyelohexan-1-formyl (MCC), N-succinimidyl(4-iodoacetyl)aminobenzoate (SIAB), and 6-maleimidocaproyl-valine-citrulline-p-aminobenzyloxycarbonyl (MC-vc-PAB);
preferably, the linker is 6-maleimidocaproyl-valinc-citrulline-p-aminobenzyloxycarbonyl (MC-vc-PAB).

**11.** The antibody drug conjugate of claim 1, its pharmaceutically acceptable salt, solvate or solvate of said salt, wherein:

Ab includes:

(a) the heavy chain variable region CDR1, CDR2, CDR3 and the light chain variable region CDR1, CDR2, CDR3, wherein, the sequence of the heavy chain variable region CDR1 is shown in SEQ ID NO: 42, the sequence of the heavy chain variable region CDR2 is shown in SEQ ID NO: 43, the sequence of the heavy chain variable region CDR3 is shown in SEQ ID NO: 44, the sequence of the light chain variable region CDR1 is shown in SEQ ID NO: 50, the sequence of the light chain variable region CDR2 is shown in SEQ ID NO: 51, and the sequence of the light chain variable region CDR3 is shown in SEQ ID NO: 52;
(b) a heavy chain variable region and a light chain variable region, wherein the sequence of the heavy chain variable region is shown in SEQ ID NO: 41, and the sequence of the light chain variable region is shown in SEQ ID NO: 49; and /or
(c) heavy chain and light chain, wherein the sequence of the heavy chain is shown in SEQ ID NO: 65, and the sequence of the light chain is shown in SEQ ID NO: 66;

L is MC-vc-PAB; and
D is MMAE.

**12.** A composition comprising the antibody drug conjugate of any one of claims 1-11, its pharmaceutically acceptable salt, solvate or solvate of said salt; optionally, further comprising at least one of the chemotherapeutic drugs, immunotherapy drugs and immunosuppressants known for treating tumors; or optionally, at least one pharmaceutically acceptable carrier, diluent or excipient.

**13.** Use of the antibody drug conjugate of any one of claims 1-11, its pharmaceutically acceptable salt, solvate or solvate of said salt or the composition of claim 12 in the preparation of medicaments for preventing and/or treating a disease associated with Claudin 18.2;

preferably, the disease associated with Claudin 18.2 is gastric cancer, adenocarcinoma of the esophagogastric junction, and pancreatic cancer;
more preferably, the disease associated with Claudin 18.2 is gastric cancer.

**14.** A method for preventing and/or treating a disease associated with Claudin 18.2, comprising: administering to a subject in need a prophylactically and/or therapeutically effective amount of the antibody drug conjugate of any one of claims 1-11, its pharmaceutically acceptable salt, solvate or solvate of said salt, or the composition of claim 12;

preferably, the disease associated with Claudin 18.2 is gastric cancer, adenocarcinoma of the esophagogastric

junction, and pancreatic cancer;
more preferably, the disease associated with Claudin 18.2 is gastric cancer.

**15.** The antibody drug conjugate of any one of claims 1-11, its pharmaceutically acceptable salt, solvate or solvate of said salt, or the composition of claim 12, for use in preventing and/or treating a disease associated with Claudin 18.2;

preferably, the disease associated with Claudin 18.2 is gastric cancer, adenocarcinoma of the esophagogastric junction, and pancreatic cancer;
more preferably, the disease associated with Claudin 18.2 is gastric cancer.

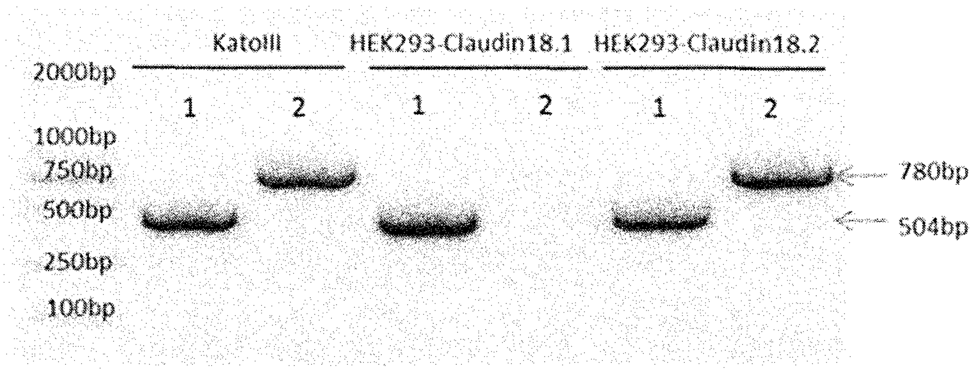
KatoIII
HEK293-Claudin18.1
HEK293-Claudin18.2
2000bp
1000bp
750bp
500bp
250bp
100bp
1
2
1
2
1
2
780bp
504bp

Fig. 1

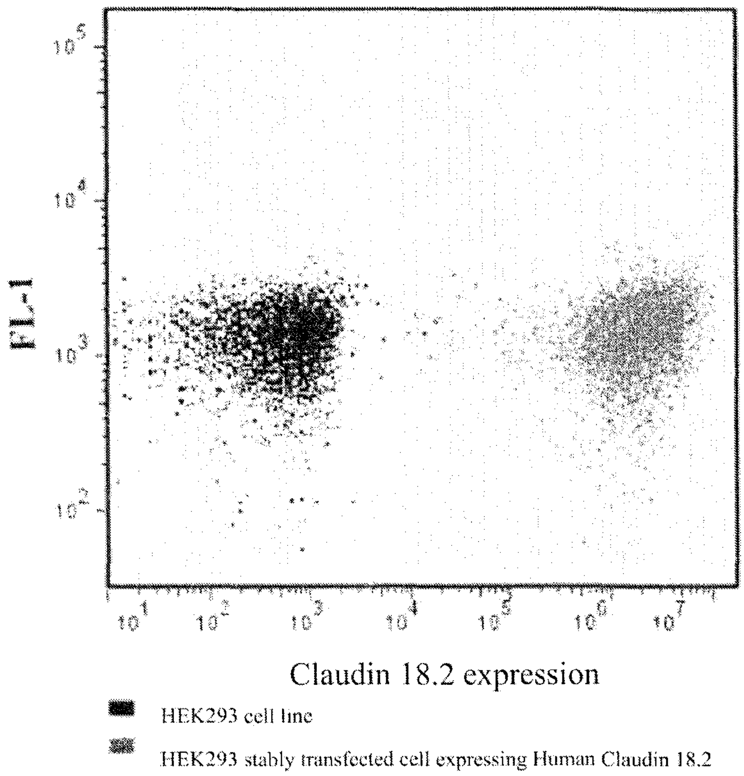
FL-1
$10^5$
$10^4$
$10^3$
$10^2$
$10^1$
$10^2$
$10^3$
$10^4$
$10^5$
$10^6$
$10^7$
Claudin 18.2 expression
HEK293 cell line
HEK293 stably transfected cell expressing Human Claudin 18.2

Fig. 2

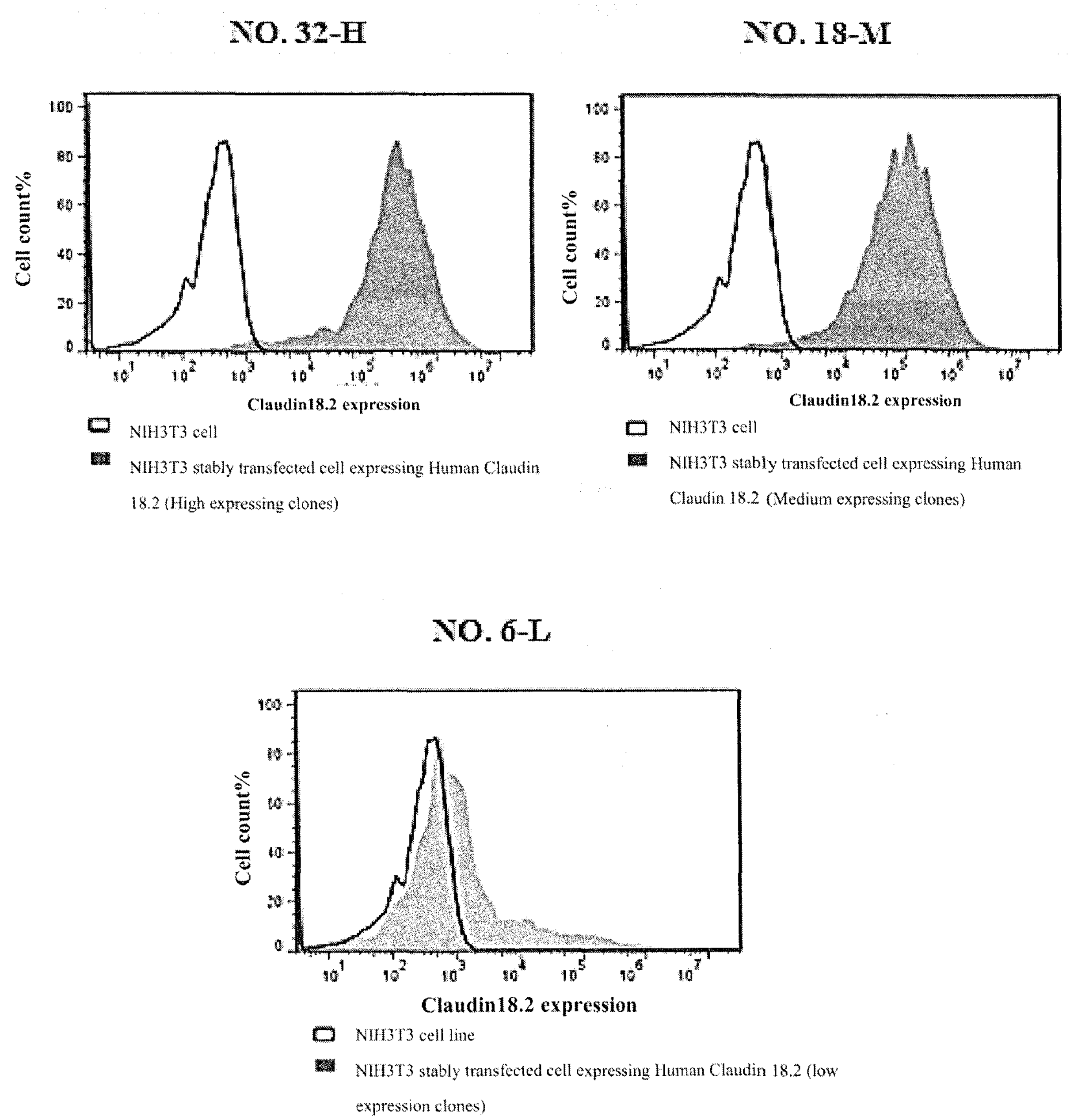
NO. 32-H
Cell count%
Claudin18.2 expression
NIH3T3 cell
NIH3T3 stably transfected cell expressing Human Claudin 18.2 (High expressing clones)
NO. 18-M
Cell count%
Claudin18.2 expression
NIH3T3 cell
NIH3T3 stably transfected cell expressing Human Claudin 18.2 (Medium expressing clones)
NO. 6-L
Cell count%
Claudin18.2 expression
NIH3T3 cell line
NIH3T3 stably transfected cell expressing Human Claudin 18.2 (low expression clones)

Fig. 3

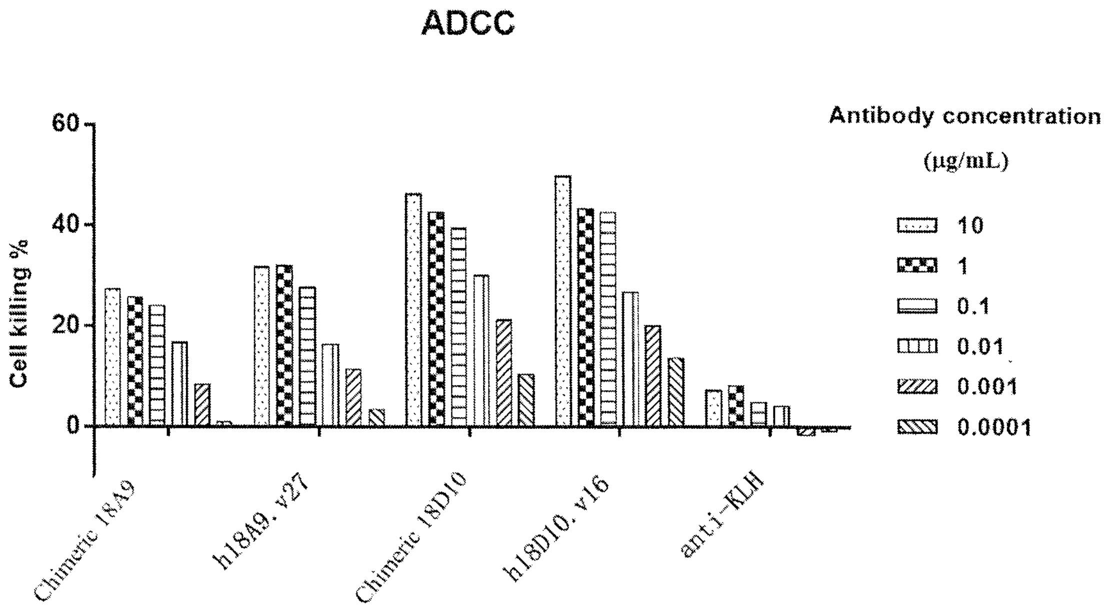
ADCC
Antibody concentration
(μg/mL)
10
1
0.1
0.01
0.001
0.0001
Cell killing %
60
40
20
0
Chimeric 18A9
h18A9.v27
Chimeric 18D10
h18D10.v16
anti-KLH

Fig. 4

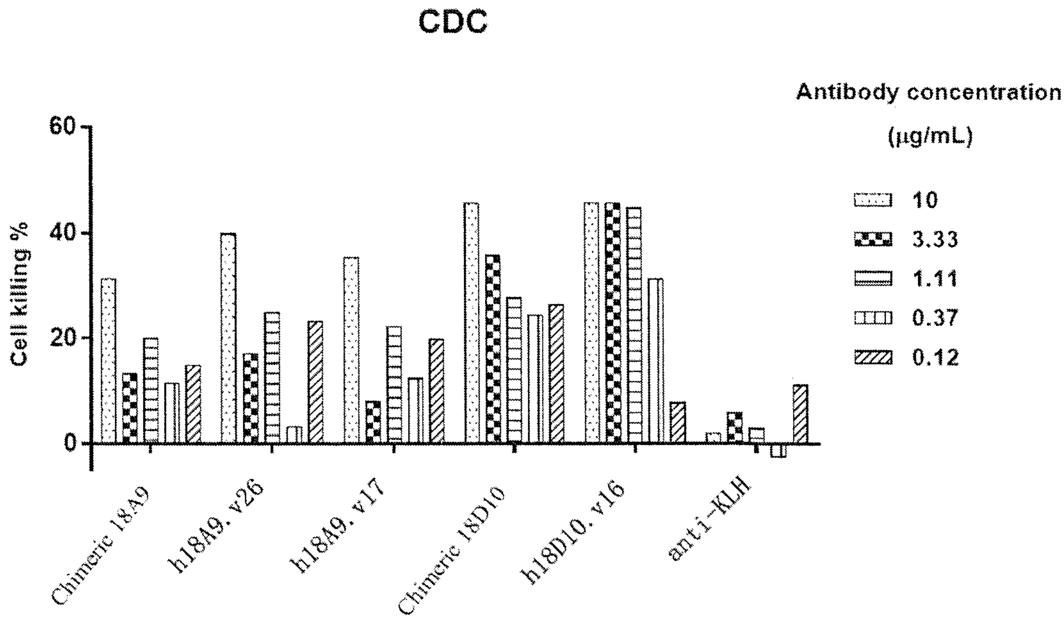
CDC
Antibody concentration
(μg/mL)
10
3.33
1.11
0.37
0.12
Cell killing %
60
40
20
0
Chimeric 18A9
h18A9.v26
h18A9.v17
Chimeric 18D10
h18D10.v16
anti-KLH

Fig. 5

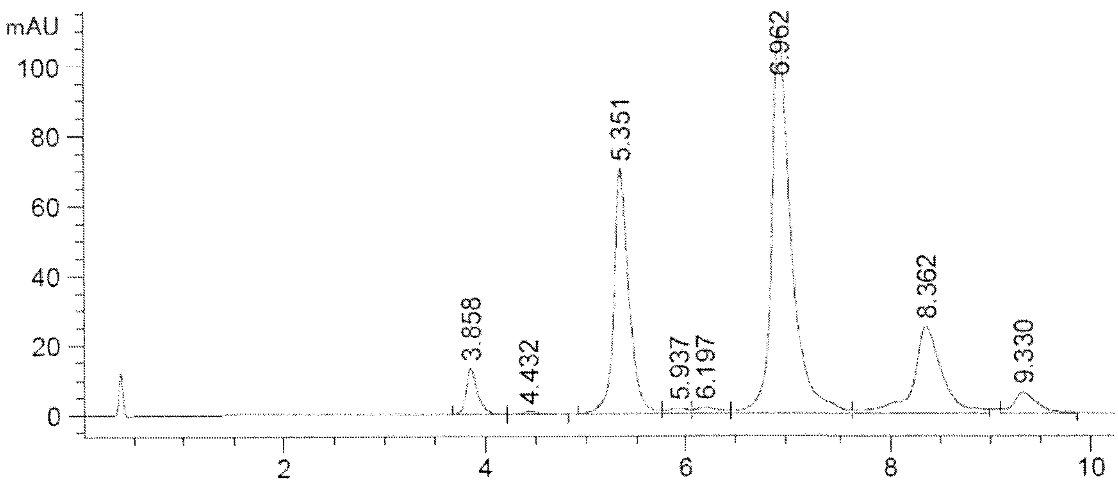
mAU
100
80
60
40
20
0
3.858
4.432
5.351
5.937
6.197
6.962
8.362
9.330
2
4
6
8
10

Fig. 6

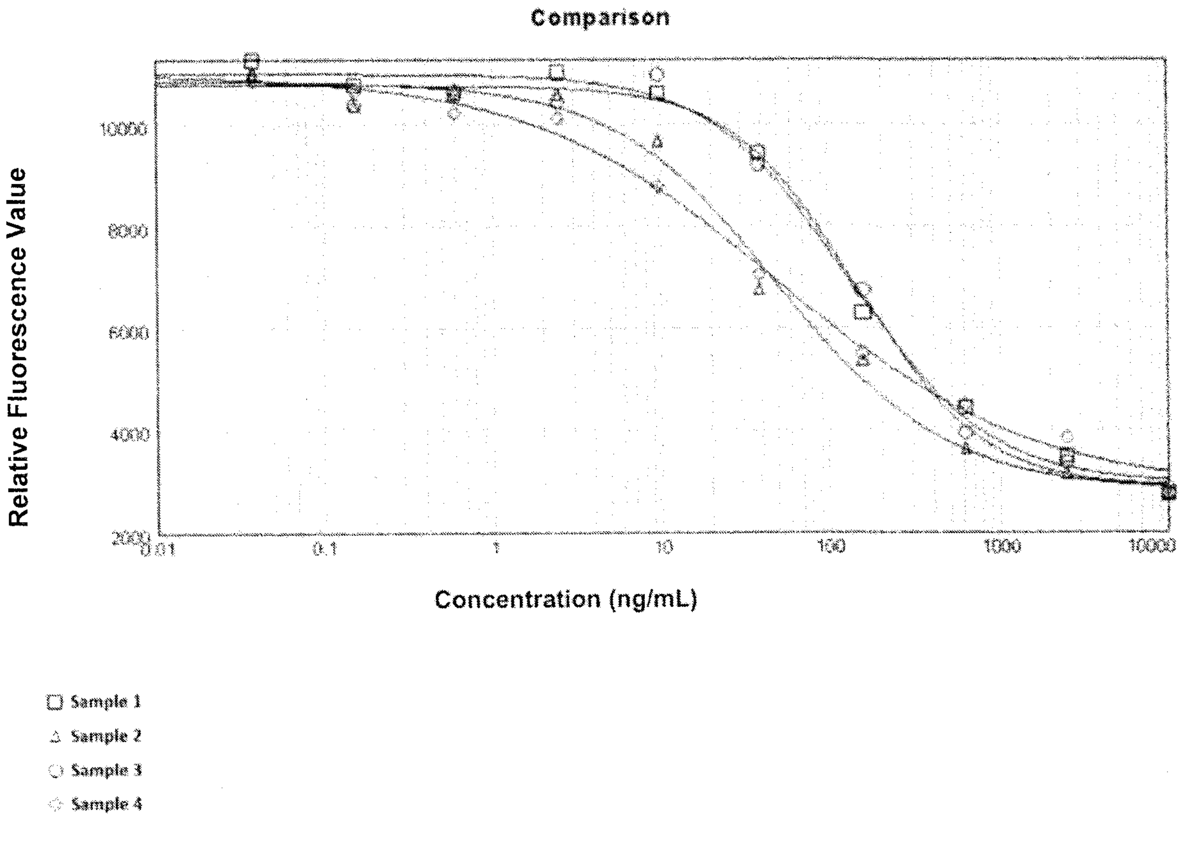
Comparison
Relative Fluorescence Value
10000
8000
6000
4000
2000
0.01
0.1
1
10
100
1000
10000
Concentration (ng/mL)
Sample 1
Sample 2
Sample 3
Sample 4

Fig. 7

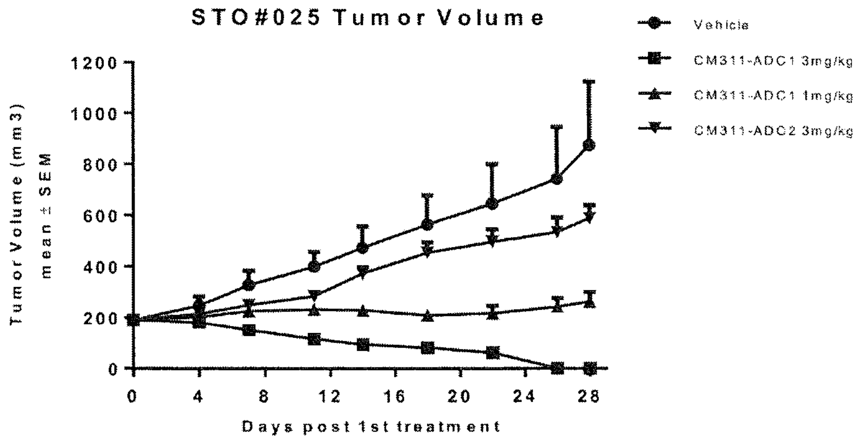


Fig. 8

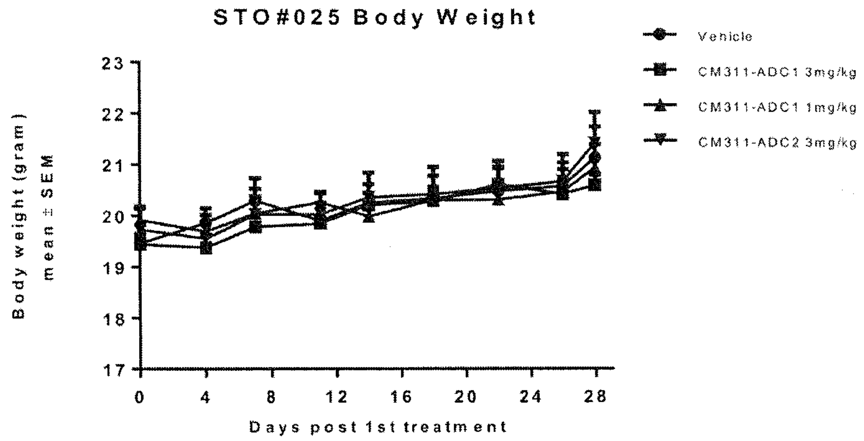


Fig. 9

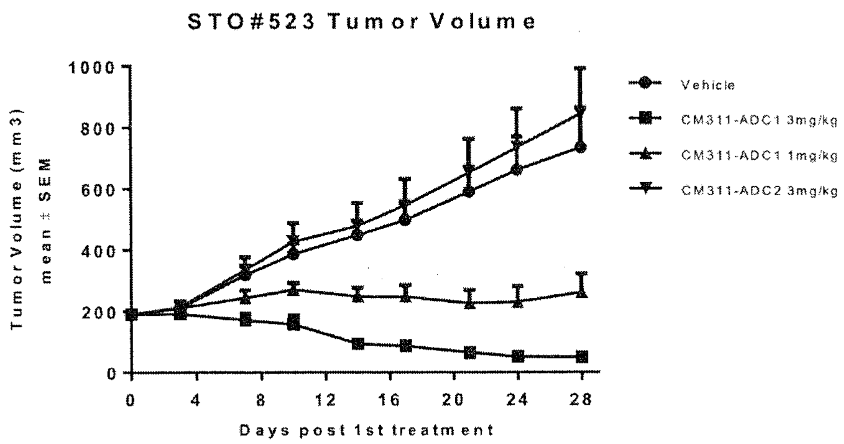


Fig. 10

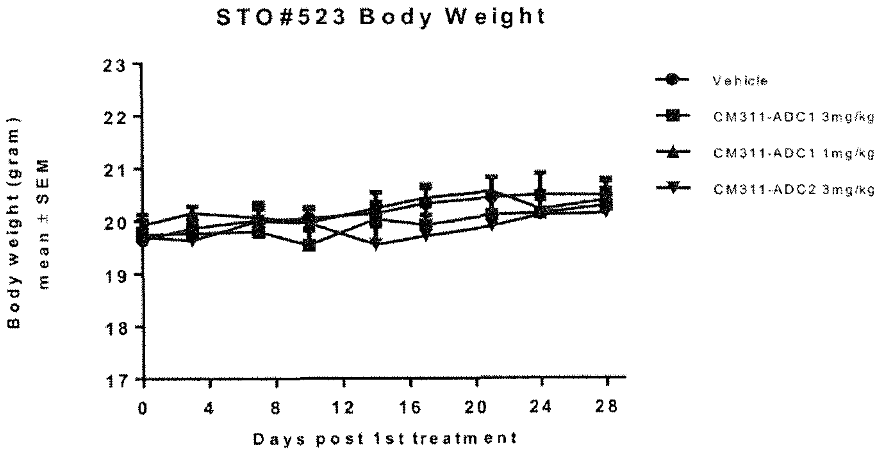
STO#523 Body Weight
Body weight (gram)
mean ± SEM
23
22
21
20
19
18
17
0
4
8
12
16
20
24
28
Days post 1st treatment
Vehicle
CM311-ADC1 3mg/kg
CM311-ADC1 1mg/kg
CM311-ADC2 3mg/kg

Fig. 11

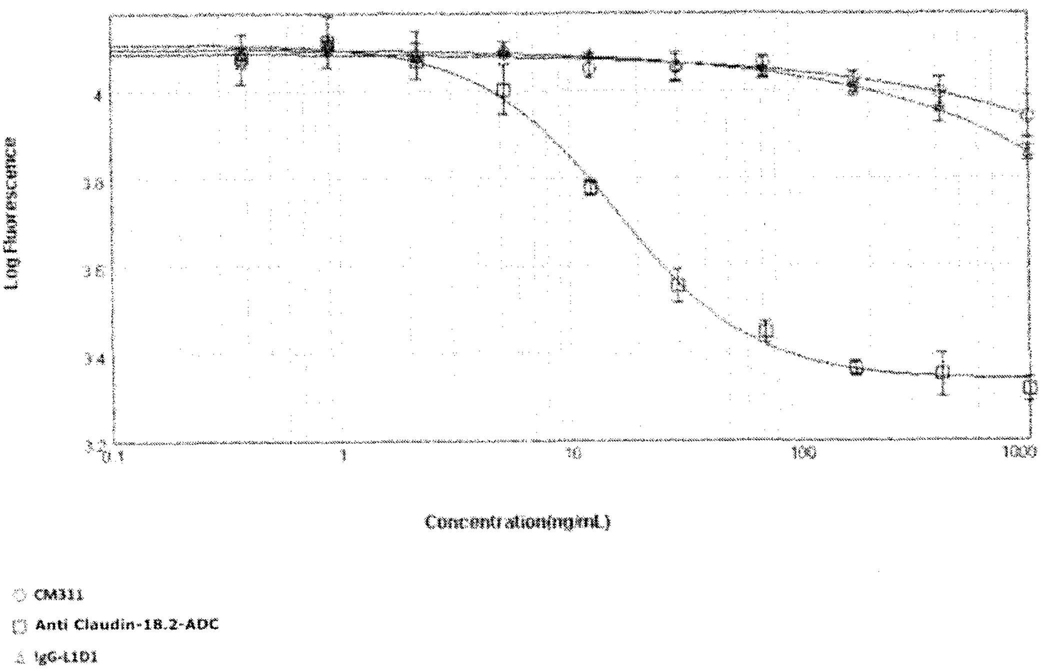
Log Fluorescence
4
3.8
3.6
3.4
0.1
1
10
100
1000
Concentration(ng/mL)
CM311
Anti Claudin-18.2-ADC
IgG-L1D1

Fig. 12

**INTERNATIONAL SEARCH REPORT**

International application No. **PCT/CN2021/124698**

**A. CLASSIFICATION OF SUBJECT MATTER**

A61K 47/68(2017.01)i; A61K 39/395(2006.01)i; C07K 16/18(2006.01)i; C07K 16/30(2006.01)i; A61P 35/00(2006.01)i

According to International Patent Classification (IPC) or to both national classification and IPC

**B. FIELDS SEARCHED**

Minimum documentation searched (classification system followed by classification symbols)

A61K47/-, A61K39/-, C07K16/-, A61P35/-

Documentation searched other than minimum documentation to the extent that such documents are included in the fields searched

Electronic data base consulted during the international search (name of data base and, where practicable, search terms used)

CNABS, CNTXT, CNKI, 百度, 百度学术, VEN, DWPI, SIPOABS, WOTXT, USTXT, EPTXT, JPTXT, ISI-WEB OF SCIENCE, STN: 上海美雅珂生物技术有限责任公司, 康诺亚生物医药科技(成都)有限公司, 胡朝红, 李虎, 陈博, 徐刚, 王莹, 紧密连接蛋白, 密蛋白, Claudin18, CLDN18, sequence SEQ ID NO: 1-8, 49-56, 182, 188

**C. DOCUMENTS CONSIDERED TO BE RELEVANT**

| Category* | Citation of document, with indication, where appropriate, of the relevant passages | Relevant to claim No. |
|---|---|---|
| PX | WO 2020211792 A1 (CHENGDU CONMED BIOSCIENCES CO., LTD) 22 October 2020 (2020-10-22)<br>description page 1 lines 19-29, page 7 lines 4-6 from the bottom, page 8 lines 31-35, page 10 lines 5-6 | 1-4, 6, 8-10, 12-13, 15 |
| A | CN 111110862 A (L&L BIOPHARMA, CO., LTD.) 08 May 2020 (2020-05-08)<br>entire document | 1-4, 6, 8-10, 12-13, 15 |
| A | WO 2016165762 A1 (GANYMED PHARMACEUTICALS AG et al.) 20 October 2016 (2016-10-20)<br>entire document | 1-4, 6, 8-10, 12-13, 15 |
| A | CN 109810039 A (SHANGHAI QINGRUN PHARMACEUTICAL TECHNOLOGY CO., LTD.) 28 May 2019 (2019-05-28)<br>entire document | 1-4, 6, 8-10, 12-13, 15 |
| A | ZHU G. Y. et al. "Targeting CLDN18.2 by CD3 Bispecifc and ADC Modalities for the Treatments of Gastric and pancreatic Cancer"<br>*SCIENTIFIC REPORTS,* Vol. 9, 10 June 2019 (2019-06-10),<br>ISSN: 2045-2322,<br>p. 8420 | 1-4, 6, 8-10, 12-13, 15 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **21 December 2021** | **18 January 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No.
**PCT/CN2021/124698**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

INTERNATIONAL SEARCH REPORT

International application No.
PCT/CN2021/124698

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **14**
because they relate to subject matter not required to be searched by this Authority, namely:

[1] Claim 14 sets forth a method for preventing and/or treating diseases associated with Claudin 18.2, belongs to methods for treating the human or animal body, and belongs to subject matter for which a search is not required under PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

[1] According to permutation and combination of CDR sequences in claim 1, claims 1-15 are divided into 140608 inventions, detailed as follows:

[2] 1) A portion of the technical solutions of claims 1-4, 6, 8-10 and 12-15: relate to an antibody drug conjugate, a pharmaceutically acceptable salt, a solvate, or a solvate of said salt thereof. Said antibody drug conjugate is with the structure of formula I, Ab-(L-D)p, wherein: Ab is an anti-Claudin 18.2 antibody, which comprises a heavy chain and a light chain, the heavy chain variable region CDR1 being selected from SEQ ID NO: 2, heavy chain variable region CDR2 is selected from SEQ ID NO: 3, heavy chain variable region CDR3 is selected from SEQ ID NO: 4, the light chain variable region CDR1 being selected from SEQ ID NO: 50, the light chain variable region CDR2 being selected from SEQ ID NO: 51, and the light chain variable region CDR3 being selected from SEQ ID NO: 52; D is a cytotoxic agent; L is a joint used to link said anti-Claudin 18.2 antibody and said cytotoxic agent; and p is 2.0-8.0;

[3] 2) A portion of the technical solutions of claims 1-4, 6, 8-10 and 12-15: relate to an antibody drug conjugate, a pharmaceutically acceptable salt, a solvate, or a solvate of said salt thereof. Said antibody drug conjugate has the structure of formula I, Ab-(L-D)p, comprising: Ab is an anti-Claudin 18.2 antibody, which comprises a heavy chain and a light chain, the heavy chain variable region CDR1 being selected from SEQ ID NO: 2, the heavy chain variable region CDR2 being selected from SEQ ID NO: 11, the heavy chain variable region CDR3 being selected from SEQ ID NO: 4, the light chain variable region CDR1 being selected from SEQ ID NO: 50, the light chain variable region CDR2 being selected from SEQ ID NO: 51, and the light chain variable region CDR3 being selected from SEQ ID NO: 52; D is a cytotoxic agent; L is a joint used to link said anti-Claudin 18.2 antibody and said cytotoxic agent; and p is 2.0-8.0;

[4] n) A portion of the technical solutions of claims 1-15: relate to an antibody drug conjugate, a pharmaceutically acceptable salt, a solvate, or a solvate of said salt thereof. Said antibody drug conjugate has the structure of formula I, Ab-(L-D)p, comprising: Ab is an anti-Claudin 18.2 antibody, which comprises a heavy chain and a light chain, the heavy chain variable region CDR1 being selected from SEQ ID NO: 42, the heavy chain variable region CDR2 being selected from SEQ ID NO: 43, the heavy chain variable region CDR3 being selected from SEQ ID NO: 44, the light chain variable region CDR1 being selected from SEQ ID NO: 50, the light chain variable region CDR2 ibeing selected from SEQ ID NO: 51, and the light chain variable region CDR3 being selected from SEQ ID NO: 52; D is a cytotoxic agent; L is a joint used to link said anti-Claudin 18.2 antibody and said cytotoxic agent; and p is 2.0-8.0;

[5] ……

[6] 140608): A portion of the technical solutions of claims 1-4, 6, 8-10 and12-15: relate to an antibody drug conjugate, a pharmaceutically acceptable salt, a solvate, or a solvate of said salt thereof. Said antibody drug

Form PCT/ISA/210 (continuation of first sheet) (January 2015)

**INTERNATIONAL SEARCH REPORT**

International application No.
**PCT/CN2021/124698**

**Box No. III Observations where unity of invention is lacking (Continuation of item 3 of first sheet)**

conjugate has the structure of formula I, Ab-(L-D)p, comprising: Ab is an anti-Claudin 18.2 antibody, which comprises a heavy chain and a light chain, the heavy chain variable region CDR1 being selected from SEQ ID NO: 116, the heavy chain variable region CDR2 being selected from SEQ ID NO: 117, the heavy chain variable region CDR3 being selected from SEQ ID NO: 118, the light chain variable region CDR1 being selected from SEQ ID NO: 132, the light chain variable region CDR2 being selected from SEQ ID NO: 133, and the light chain variable region CDR3 being selected from SEQ ID NO: 134; D is a cytotoxic agent; L is a joint used to link said anti-Claudin 18.2 antibody and said cytotoxic agent; and p is 2.0-8.0.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **1-4,6,8-10,12-15**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
**Information on patent family members**

International application No.
**PCT/CN2021/124698**

| Patent document cited in search report | Publication date (day/month/year) | Patent family member(s) | Publication date (day/month/year) |
|---|---|---|---|
| WO 2020211792 A1 | 22 October 2020 | None | |
| CN 111110862 A | 08 May 2020 | EP 3808376 A4 | 01 September 2021 |
| | | EP 3808376 A1 | 21 April 2021 |
| | | US 20210230272 A1 | 29 July 2021 |
| | | CN 110606891 A | 24 December 2019 |
| | | WO 2019242505 A1 | 26 December 2019 |
| | | CN 111867630 A | 30 October 2020 |
| | | CN 111848809 A | 30 October 2020 |
| | | CN 111518214 A | 11 August 2020 |
| WO 2016165762 A1 | 20 October 2016 | SG 10202105142 A1 | 29 June 2021 |
| | | ZA 201705923 A | 26 June 2019 |
| CN 109810039 A | 28 May 2019 | CA 3044898 A1 | 31 May 2018 |
| | | JP 2020510677 A | 09 April 2020 |
| | | US 10987430 B2 | 27 April 2021 |
| | | WO 2018095422 A1 | 31 May 2018 |
| | | CN 110088086 A | 02 August 2019 |
| | | EP 3546448 A1 | 02 October 2019 |
| | | EP 3546448 A4 | 01 July 2020 |
| | | CN 108101825 A | 01 June 2018 |
| | | KR 20190085539 A | 18 July 2019 |
| | | US 2019388555 A1 | 26 December 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **KABAT.** Sequences of Proteins of Immunological Interest. National Institutes of Health, Bethesda, 1987
- **CHOTHIA ; LESK.** *J. Mol. Biol,* 1987, vol. 196, 901-917
- **CHOTHIA et al.** *Nature,* 1989, vol. 342, 878-883
- **ALTSCHUL et al.** *Nucl. Acid. Res,* 1977, vol. 25, 3389-3402
- **ALTSCHUL et al.** *J. Mol. Biol.,* 1990, vol. 215, 403-410
- Burger's Medicinal Chemistry and Drug Discovery. 1995, vol. 172-178, 949-982
- REMINGTON'S PHARMACEUTICAL SCIENCES. Mack Publishing Company, 1995
- Immunology-A Synthesis. Sinauer Associates, 1991